# EUROPEAN PATENT APPLICATION

(11) **EP 4 403 134 A1**
(43) Date of publication of application: **24.07.2024**
(21) Application number: 23152718.5
(22) Date of filing: 20.01.2023
(51) Int. Cl.: A61F 2/06, A61F 2/82, A61F 2/02

(54) **DEVICE FOR ENDOVASCULAR DRUG DELIVERY**

(71) Applicant: Julius-Maximilians-Universität Würzburg, 97070 Würzburg (DE)
(72) Inventor: The designation of the inventor has not yet been filed
(74) Representative: Engelhard, Markus

(57) **Abstract**

The present invention relates to a device for endovascular drug delivery, preferably an implant for endovascular drug delivery. The present invention further relates to a device for use in a method of preventing or treating a neurological disease, an oncological disease, a cardiological disease, a vascular disease, an immunological disease, a carcinoid, an infectious disease, and/or an edema.

## Description

### FIELD OF THE INVENTION

The present invention relates to a device for endovascular drug delivery, preferably an implant for endovascular drug delivery. The present invention further relates to a device for use in a method of preventing or treating a neurological disease, an oncological disease, a cardiological disease, a vascular disease, an immunological disease, a carcinoid, an infectious disease, an edema, and/ or any disease which can be treated by angiography.

### BACKGROUND OF THE INVENTION

Currently, pharmaceuticals are typically applied orally or intravenously, and reach their target via the circulatory system. However, when systemically applied, the pharmaceuticals may also reach off-targets, such as non-target tissues, thereby causing side effects. For example, in chemotherapy, the desired effects on the tumor tissue are typically accompanied by undesired cytotoxic effects in the area of hair follicles, intestinal and oral mucous membranes, and/or the bone marrow. These off-target effects can result in burdening and possibly life-threatening side effects. In some cases the side effects are so severe that the oncologic therapy needs to be terminated. A further disadvantage of a systemic application of active agents is that the active agents are diluted in the blood circulation and thus high dosages of the active agents may be required to achieve a therapeutic effect.

Off-target effects of applied pharmaceuticals are also a major challenge in the treatment of other conditions, such as neurological conditions, e.g. in post-treatment of ischemic strokes. During an ischemic stroke, a thrombus blocks the blood flow in a blood vessel of the brain. Depending on the size of the thrombus, the thrombus needs to be removed by mechanical thrombectomy with a stent retriever. Despite a successful thrombectomy, the formation of a cerebral edema -can lead to irreversible brain damage. Conventionally, anti-inflammatory glucocorticoids are systemically applied to treat diseases such as brain cancer. Recent findings on the immune reaction in the brain have led to the development of new therapy approaches for treating inflammatory brain conditions, such as stroke cerebral edema. However, many active agents with the potential to protect brain cells after ischemic strokes cannot be applied in sufficient dosages due to severe systemic side effects. For example, the proteasome inhibitor bortezomib, which is approved for treatment of multiple myeloma, is a possible candidate for post-treatment of ischemic strokes. However, when systemically applied, bortezomib may cause brain hemorrhages by inducing thrombocytopenia by the inhibition of proplatelet formation of megakaryocytes. The presently available treatment options for treating diseases such as cerebral edema after a stroke are insufficient. Particularly, the present treatment options are insufficient with respect to the active agent crossing of the blood-brain barrier and/or side effects.

Thus, there is a need for enhanced therapeutic options. Particularly, there is a need for devices that allow to apply active agents in a localized manner. Furthermore, there is a need for devices that can locally release active agents, particularly in a controlled and optionally sequential manner. There is also the need for devices which allow to release active agents in a sustained manner. Furthermore, there is the need for devices that allow an enhanced treatment of diseases such as a neurological disease, an oncological disease, a cardiological disease, a vascular disease, an immunological disease, a carcinoid, an infectious disease, and/or an edema. There is also the need for enhanced endovascular drug delivery devices. Moreover, there remains the need for enhanced means for treating diseases such as cerebral edema associated with stroke. There also remains the need for enhanced devices allowing to reduce dosages of active agents.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a device for endovascular drug delivery, preferably an implant for endovascular drug delivery,
wherein said device has a substantially cylindrical form;
wherein said device comprises a channel, preferably an internal channel; wherein, preferably, said channel is configured to allow blood flow through said device;
wherein said device is preferably elastically deformable, preferably elastically deformable in a direction substantially perpendicular to a longitudinal axis of said device;
wherein said device optionally comprises a biocompatible polymeric matrix comprising a biocompatible polymer; and
wherein said device comprises an active agent;
wherein, preferably, said device is biodegradable;
wherein, preferably, said device is configured to release said active agent into said channel.

In one embodiment, said biocompatible polymer is selected from poly(lactic-co-glycolic acid), poly(lactic acid), polycaprolactone, poly(glycolic acid), poly(glycerol sebacate), polycaprolactam, elastin, fibrillin, fibrullin, poly(1-acrylonitrile), poly(vinyl alcohol), polygluconate, polyglycolide, dextran, type I collagen, type II collagen, type IV collagen, elastin, silk fibroin, polymandelide, poly(trimethylene carbonate), polydioxanone, poly(4-hydroxybutyrate), poly(butylene succinate), polyphosphazenes, polyanhydrides, polyphosphoesters, polyoxalate, silica gel, alginate, polyethylene glycol, poly(2-oxazoline), gelatin, polyglycidol, polyurethane, and combinations thereof;
wherein, preferably, said biocompatible polymer is selected from poly(lactic-co-glycolic acid), poly(lactic acid), polycaprolactone, poly(glycolic acid), poly(glycerol sebacate), and combinations thereof;
wherein, more preferably, said biocompatible polymer is poly(lactic-co-glycolic acid), polycaprolactone, or a combination thereof.

In one embodiment, said device comprises fibers, preferably nanofibers and/or microfibers, such as polymeric nanofibers; wherein, optionally, said fibers, preferably polymeric nanofibers, comprise silicon dioxide and/or titanium oxide;
wherein, preferably, said device comprises nanofibers, preferably selected from poly(lactic-co-glycolic acid) nanofibers, polycaprolactone nanofibers, poly(lactic acid) nanofibers, poly(lactic acid) nanofibers comprising titanium oxide, silica gel nanofibers, and combinations thereof.

In one embodiment, said fibers are aligned along a longitudinal axis of said device; said fibers are aligned at an angle in a range of from >0° to <180° to the longitudinal axis of said device; said fibers are aligned along a circumferential direction of said device; said fibers are aligned along a radial direction of said device; said fibers are oriented as a grid; and/or said fibers are randomly oriented.

In one embodiment, said device comprises nanoparticles, preferably mesoporous silica nanoparticles, poly(lactic-co-glycolic acid) nanoparticles, and/or inulin nanoparticles.

In one embodiment, said device comprises a hydrogel;
wherein, optionally, said hydrogel is configured to release said active agent, preferably to release said active agent into said channel, if said hydrogel is contacted with a fluid, particularly blood.

In one embodiment, said device comprises a first layer and a second layer; optionally comprises a third layer;
wherein said first layer comprises a first biocompatible polymer, preferably a shape memory polymer; wherein, optionally, said first layer comprises nanofibers, nanoparticles, a hydrogel, an active agent, and/or a cell adhesion molecule;
wherein said second layer comprises a second biocompatible polymer, preferably poly(lactic-co-glycolic acid) or polycaprolactone; wherein, optionally, said second layer comprises nanofibers, nanoparticles, a hydrogel, and/or an active agent;
wherein said third layer, if present, comprises said active agent, optionally comprises a third biocompatible polymer, nanofibers, nanoparticles, and/or a hydrogel;
wherein at least one of said first layer, said second layer, and, if present, said third layer, comprises said active agent.

In one embodiment, said device comprises a first layer and a second layer; optionally comprises a third layer;
wherein said first layer comprises a first biocompatible polymer, preferably a shape memory polymer; wherein, optionally, said first layer comprises fibers, nanoparticles, a hydrogel, an active agent, and/or a cell adhesion molecule;
wherein said second layer comprises a second biocompatible polymer, preferably poly(lactic-co-glycolic acid) or polycaprolactone; wherein, optionally, said second layer comprises fibers, nanoparticles, a hydrogel, and/or an active agent;
wherein said third layer, if present, comprises said active agent, optionally comprises a third biocompatible polymer, fibers, nanoparticles, and/or a hydrogel;
wherein at least one of said first layer, said second layer, and, if present, said third layer, comprises said active agent. In one embodiment, said fibers are nanofibers and/or microfibers.

In one embodiment, said device comprises a first layer, a second layer, and a third layer;
wherein said first layer comprises a first biocompatible polymer, preferably a shape memory polymer;
wherein said second layer comprises a second biocompatible polymer, preferably poly(lactic-co-glycolic acid) or polycaprolactone, and an active agent such as an immunosuppressant;
wherein, optionally, said second layer comprises nanofibers, nanoparticles, and/or a hydrogel; wherein, preferably, said second layer comprises nanoparticles comprising said active agent;
wherein said third layer comprises a third biocompatible polymer, preferably poly(lactic-co-glycolic acid) or polycaprolactone, and an active agent such as a chemotherapeutic agent;
wherein, optionally, said third layer comprises nanofibers, nanoparticles, and/or a hydrogel;
wherein, preferably, said third layer comprises nanoparticles comprising said active agent;
wherein, optionally, a density of said third layer is lower than a density of said second layer;
wherein, optionally, the molar mass of said third layer is lower than the molar mass of said second layer;
wherein, optionally, said active agent of said second layer is different from said active agent of said third layer.

In one embodiment, said device comprises a first layer, a second layer, and a third layer;
wherein said first layer comprises a first biocompatible polymer, preferably a shape memory polymer;
wherein said second layer comprises a second biocompatible polymer, preferably poly(lactic-co-glycolic acid) or polycaprolactone, and an active agent such as an immunosuppressant; wherein, optionally, said second layer comprises fibers, nanoparticles, and/or a hydrogel; wherein, preferably, said second layer comprises nanoparticles comprising said active agent;
wherein said third layer comprises a third biocompatible polymer, preferably poly(lactic-co-glycolic acid) or polycaprolactone, and an active agent such as a chemotherapeutic agent; wherein, optionally, said third layer comprises fibers, nanoparticles, and/or a hydrogel; wherein, preferably, said third layer comprises nanoparticles comprising said active agent;
wherein, optionally, a density of said third layer is lower than a density of said second layer;
wherein, optionally, the molar mass of said third layer is lower than the molar mass of said second layer;
wherein, optionally, said active agent of said second layer is different from said active agent of said third layer. In one embodiment, said fibers are nanofibers and/or microfibers.

In one embodiment, said cylindrical form is a hollow cylindrical form and/or an open cylindrical form.

In one embodiment, said device has
a length in a range of from about 0.25 mm to about 80 mm, preferably in a range of from about 0.5 mm to about 50 mm, more preferably in a range of from about 1 mm to about 25 mm, even more preferably in a range of from about 3 mm to about 20 mm,
an outer radius in a range of from about 2 µm to about 6 mm, preferably in a range of from about 0.5 mm to about 4 mm, more preferably in a range of from about 1 mm to about 3.5 mm, even more preferably in a range of from about 1.4 mm to about 3 mm,
an inner radius in a range of from about 1 µm to about 5.9 mm, preferably in a range of from about 0.4 mm to about 3.9 mm, more preferably in a range of from about 0.9 mm to about 3.4 mm, even more preferably in a range of from about 1.3 mm to about 3 mm, and/or
a thickness in a range of from about 1 µm to about 1 mm, preferably in a range of from about 20 µm to about 500 µm, preferably in a range of from about 50 µm to about 250 µm, even more preferably in a range of from about 75 µm to about 150 µm, e.g. about 100 µm.

In one embodiment, said device comprises a cutting line, optionally a cutting line along a longitudinal axis of said device.

In one embodiment, said device is biodegradable, preferably bioresorbable,
wherein, optionally, said device is biodegradable, preferably bioresorbable, at a temperature in a range of from about 34°C to about 43°C.

In one embodiment, said active agent is embedded in said polymeric matrix, optionally is embedded in a hydrogel present in said polymeric matrix and/or is encapsulated in nanoparticles present in said polymeric matrix; said active agent is bound to said device, preferably to an inner surface of said device, preferably via a linker; and/or said active agent is coated onto an inner surface of said device, preferably onto a surface of said channel.

In one embodiment, said active agent is selected from chemotherapeutic agents such as proteasome inhibitors, e.g. bortezomib, or anthracyclines, e.g. doxorubicin; immunosuppressants such as glucocorticoids, e.g. dexamethasone; calcium channel blockers such as nimodipine; antithrombotic agents such as tissue plasminogen activator, acetylsalicylic acid, phenprocoumon, dabigatran, apixaban, edoxaban, or heparin; antibodies such as anti-ICAM-1 antibodies, anti-MCH2 antibodies, or anti-VEGF antibodies; antihypertensive agents such as angiotensin-converting-enzyme inhibitors or beta blockers; matrix metalloproteinase inhibitors; cytokines such as interleukins; chemokines; Parkinson's disease drugs such as levodopa, dopamine agonists, or monoamine oxidase-B inhibitors; Huntington's disease drugs such as tetrabenazine, deutetrabenazine, haloperidol, fluphenazine, amantadine, levetiracetam, or clonazepam; and combinations thereof;
wherein, preferably, said active agent is selected from chemotherapeutic agents such as proteasome inhibitors, e.g. bortezomib; immunosuppressants such as glucocorticoids, e.g. dexamethasone; calcium channel blockers such as nimodipine; antibodies such as anti-ICAM-1 antibodies, anti-MCH2 antibodies, or anti-VEGF antibodies; and combinations thereof; wherein, optionally, said active agent is selected from bortezomib, dexamethasone, nimodipine, an anti-ICAM-1 antibody, and combinations thereof;
wherein, more preferably, said active agent is selected from chemotherapeutic agents, preferably proteasome inhibitors, e.g. bortezomib.

In a further aspect, the present invention relates to a kit comprising a device, as defined herein, and a protective cover, preferably a protective sheathing; wherein, optionally, said protective cover comprises an active agent and/or saline such as cold saline. In one embodiment, cold saline has a temperature in a range of from 1°C to 10°C, preferably of about 4°C.

In a further aspect, the present invention relates to a device, as defined herein, for use in a method of preventing or treating a neurological disease, an oncological disease, a cardiological disease, a vascular disease, an immunological disease, a carcinoid, an infectious disease, and/or an edema;
wherein, preferably, said neurological disease is a brain disease and/or neurodegenerative disease, wherein, preferably, said brain disease is selected from stroke, particularly ischemic stroke, subarachnoid hemorrhage, and basal ganglia diseases, and wherein, preferably, said neurodegenerative disease is Parkinson's disease;
wherein, preferably, said oncological disease is a cancer, preferably a solid cancer, more preferably a solid cancer selected from liver cancer, kidney cancer, colorectal cancer, osteosarcoma, pancreas cancer, brain cancer, and melanoma;
wherein, preferably, said cardiological disease is a heart disease, preferably myocardial infarction;
wherein, preferably, said vascular disease is an ischaemic disease, preferably peripheral vascular disease;
wherein, preferably, said immunological disease is graft-versus-host disease;
wherein, preferably, said edema is a brain edema.

In one embodiment, said device is administered, particularly inserted, into a blood vessel, preferably brain blood vessel, of a patient in need thereof.

In a further aspect, the present invention relates to a method of preventing or treating a neurological disease, an oncological disease, a cardiological disease, a vascular disease, an immunological disease, a carcinoid, an infectious disease, and/or an edema comprising applying, particularly administering, a device, as defined herein, to a patient in need thereof.

In one embodiment, said device is administered, particularly inserted, into a blood vessel, preferably brain blood vessel, of a patient in need thereof. In one embodiment, said neurological disease, oncological disease, cardiological disease, vascular disease, immunological disease, carcinoid, infectious disease, and/or edema are as defined herein.

In a further aspect, the present invention relates to an active agent for use in a method of preventing or treating a neurological disease, an oncological disease, a cardiological disease, a vascular disease, an immunological disease, a carcinoid, an infectious disease, and/or an edema, wherein said active agent is administered in the form or by way of a device, as defined herein, particularly by administering said device to a patient in need thereof, preferably by inserting said device into a blood vessel of a patient in need thereof;
wherein said active agent is selected from chemotherapeutic agents such as proteasome inhibitors, e.g. bortezomib, or anthracyclines, e.g. doxorubicin; immunosuppressants such as glucocorticoids, e.g. dexamethasone; calcium channel blockers such as nimodipine; antithrombotic agents such as tissue plasminogen activator, acetylsalicylic acid, phenprocoumon, dabigatran, apixaban, edoxaban, or heparin; antibodies such as anti-ICAM-1 antibodies, anti-MCH2 antibodies, or anti-VEGF antibodies; antihypertensive agents such as angiotensin-converting-enzyme inhibitors or beta blockers; matrix metalloproteinase inhibitors; cytokines such as interleukins; chemokines; Parkinson's disease drugs such as levodopa, dopamine agonists, or monoamine oxidase-B inhibitors; Huntington's disease drugs such as tetrabenazine, deutetrabenazine, haloperidol, fluphenazine, amantadine, levetiracetam, or clonazepam; and combinations thereof;
wherein, preferably, said active agent is selected from chemotherapeutic agents such as proteasome inhibitors, e.g. bortezomib; immunosuppressants such as glucocorticoids, e.g. dexamethasone; calcium channel blockers such as nimodipine; antibodies such as anti-ICAM-1 antibodies, anti-MCH2 antibodies, or anti-VEGF antibodies; and combinations thereof; wherein, optionally, said active agent is selected from bortezomib, dexamethasone, nimodipine, an anti-ICAM-1 antibody, and combinations thereof;
wherein, more preferably, said active agent is selected from chemotherapeutic agents, preferably proteasome inhibitors, e.g. bortezomib. In one embodiment, said neurological disease, oncological disease, cardiological disease, vascular disease, immunological disease, carcinoid, infectious disease, and/or edema are as defined herein.

In a further aspect, the present invention relates to the use of an active agent for the manufacture of a medicament for preventing or treating a neurological disease, an oncological disease, a cardiological disease, a vascular disease, an immunological disease, a carcinoid, an infectious disease, and/or an edema, wherein said medicament is formulated in the form of a device, as defined herein, and wherein the active agent is an active agent, as defined herein.

In one embodiment, said active agent, said neurological disease, oncological disease, cardiological disease, vascular disease, immunological disease, carcinoid, infectious disease, and/or edema are as defined herein.

### DETAILED DESCRIPTION

The present invention aims at providing drug delivery devices which reduce side effects of various active agents, particularly due to a localized administration of said active agents. Targeted and precise drug delivery at the target site prevents side effects such as adverse effects in non-target tissues. Without wishing to be bound by any theory, the inventors believe that, advantageously, due to the considerably smaller distribution volume, only a fraction of the active agent dose is required to achieve the desired active agent levels in the target tissue with a localized drug delivery using a device of the invention compared to a systemic administration. Advantageously, an implantation of the device directly at the target site allows for a better controllability and an enhanced pharmacokinetics of the released active agent(s). Particularly, since the concentration of the active agent(s) does not significantly change before reaching the target tissue, even in a case of significantly impaired kidney and/or liver function. Advantageously, at the target site, the device dissolves completely into bioresorbable components, thereby releasing the active agents. For example, more than one active agent can be sequentially released.

For example, the device of the invention can be used to prevent or treat an ischemic stroke and/or edema associated with an ischemic stroke. However, far beyond the application potential in ischemic stroke treatment and prevention, the limited crossing of the blood-brain barrier is a core problem of translational research, particularly with most active agents and for a variety of diseases such as neurological diseases. Advantageously, the device of the invention allows to shift safety-relevant dose limitations with better local penetration of the blood-brain barrier. Advantageously, with the device of the invention, locally high doses can be achieved in the target tissue while preventing side effects in distant organs.

An advantage of the device of the invention is that it can be applied with minimal invasiveness directly after a mechanical recanalization of a blood vessel with the catheter system that is already positioned in the blood vessel for the recanalization. Preferably, the device of the invention is prepared using electrospinning, centrifugal spinning, melt spinning, melt blowing, melt electrowriting, coating e.g. dip coating or spray coating, casting, inkjet printing, and/or 3D printing. For example, the device of the invention can be prepared using an electrospinning device, centrifugal spinning device, melt spinning device, melt blowing device, a melt electrowriting device, a nanocoating device, and/or a 3D printer. The advantage of melt electrowriting is that the material, e.g. the polymeric matrix, can be precisely shaped into the desired form. An advantage of electrospinning is that even heat-labile active agents, such as antibodies, can be directly incorporated in the electrospun product, e.g. the produced polymeric matrix. The device of the invention may be prepared using different techniques, such as different techniques selected from electrospinning, centrifugal spinning, melt spinning, melt blowing, melt electrowriting, coating e.g. dip coating or spray coating, casting, and 3D printing; for example, one layer of the device may be prepared by electrospinning and one layer may be prepared by electrowriting, and, optionally, any of these layers may be additionally coated using dip coating or spray coating. In one embodiment, the preparation of the device comprises coating a layer, e.g. an outer layer or inner layer, using dip coating and/or spray coating. For example, the device may be produced by electrowriting a layer, e.g. around a rotating target, and then electrospinning fibers, e.g. nanofibers and/or microfibers loaded with an active agent, around the electrowritten layer; optionally, any of the layers may be coated, for example by dip coating or spray coating.

The present invention relates to a device for endovascular drug delivery, preferably an implant for endovascular drug delivery,
wherein said device has a substantially cylindrical form;
wherein said device comprises a channel, preferably an internal channel; wherein, preferably, said channel is configured to allow blood flow through said device;
wherein said device is elastically deformable, preferably elastically deformable in a direction substantially perpendicular to a longitudinal axis of said device;
wherein said device comprises a biocompatible polymeric matrix comprising a biocompatible polymer; and
wherein said device comprises an active agent;
wherein, preferably, said device is biodegradable;
wherein, preferably, said device is configured to release said active agent into said channel.

Advantageously, the device of the invention allows to administer active agents at a target tissue, which is supplied by the terminal flow path of the blood vessel, in which the device is implanted. Thereby, target tissues can be specifically targeted and systemic side effects can be reduced.

The term "implant", as used herein, relates to a medical device manufactured to be positioned in the body of a patient. Typically, medical implants are man-made devices, in contrast to a transplant, which is a transplanted biomedical tissue. Optionally, such a device, e.g. man-made device, may comprise cells, for example human cells such as proinflammatory T-cells and/or CAR-T-cells. Preferably, the device of the invention comprises at least one layer, wherein, preferably, said at least one layer comprises or consists of said biocompatible polymeric matrix. For example, an implant for endovascular drug delivery is an implant to be positioned in a blood vessel of a patient, such as a blood vessel of a brain of a patient. Particularly, an implant for endovascular drug delivery is an implant to be positioned in a blood vessel of a patient to administer an active agent, particularly drug, to a target tissue such as brain tissue localized downstream of said device implanted in the blood vessel. Preferably, the device of the invention is configured to be positioned within a blood vessel, particularly to be positioned in the lumen surrounded by the endothelium of a blood vessel. In one embodiment, the device of the invention is configured to be positioned within a lumen of a blood vessel. In one embodiment, the device of the invention is configured to be positioned in a blood vessel of a patient and the device is not a vascular graft, particularly is not a vascular substitute, i.e. it does not replace a blood vessel such as a deficient blood vessel of a patient. The difference between the device of the invention and a vascular graft, particularly vascular substitute, is at least that it is configured to be positioned in a blood vessel instead of replacing a blood vessel. In one embodiment, the device is configured to be implanted into an intact blood vessel of a patient. The device of the invention is preferably configured to be positioned in a blood vessel. In one embodiment, the device is deployed within a blood vessel. In one embodiment, the device is configured to be deployed within a blood vessel. In one embodiment, the device has a shape and/or dimensions of a stent. In one embodiment, the device does not comprise a strut, particularly a metal strut, and/or a wire, particularly a metal wire. In a preferred embodiment, the device is sterile. The device may also be non-sterile, e.g. when provided with therapeutic cells or fragments thereof. In one embodiment, the device comprises cells, particularly therapeutic cells, or fragments thereof; wherein, preferably, said cells are therapeutic bacteria, therapeutic virus(es), immune cells such as proinflammatory T-cells, and/or CAR-T-cells. In one embodiment, the device is cell-free. In one embodiment, the device is nonimmunogenic. In one embodiment, the device is noncytotoxic. In one embodiment, the device is not ferromagnetic. Advantageously, a patient having a device of the invention, said device not being ferromagnetic, implanted in any part of the body, such as the brain, may be examined using nuclear magnetic resonance, such as cranial nuclear magnetic resonance. In one embodiment, device is configured to be positioned in a blood vessel of a patient by means of a stent retriever system and/or a balloon catheter system. In one embodiment, the device is provided with blood plasma, cytokines such as proinflammatory cytokines, and/or cells such as CAR-T-cells. For example, any of the layers of the device may be provided with blood plasma, cytokines such as proinflammatory cytokines, and/or cells such as CAR-T-cells.

In one embodiment, the device provides a unidirectional release of said active agent, particularly a release into said channel. In one embodiment, the device is configured to unidirectionally release said active agent into said channel. In one embodiment, said device is configured to release said active agent unidirectionally into said channel; wherein, preferably, said active agent is released from an inner layer and optionally subsequently from an intermediate and optionally an outer layer of said device to said channel. The term "unidirectionally", as used herein, preferably relates to the active agent being released into said channel but not into the endothelium which is contacted by an outer surface and/or an outer layer of said device. In one embodiment, the device is configured to release said active agent into the channel but not to the endothelium which is in direct contact with an outer surface and/or an outer layer of said device. For example, the device may be configured for unidirectional release by providing an inner layer of the device and optionally an intermediate layer of the device with an active agent, and by providing an outer layer of the device without an active agent.

The term "substantially cylindrical form", as used herein, relates to a form which is substantially the form of a cylinder, particularly of a cylinder being a three-dimensional solid with straight parallel sides and a circular or oval cross section. The device may have the form of a solid cylinder with circular ends perpendicular to the longitudinal axis of the cylinder. Particularly, the device may have straight parallel sides and a circular or oval cross-section. In one embodiment, said cylinder is in the form of a right cylinder or an oblique cylinder, preferably a right cylinder. In one embodiment, the device has a substantially right cylindrical form or a substantially oblique cylindrical form, preferably has a right cylindrical form. In one embodiment, the device of the invention is in the form of a substantially right circular hollow cylinder. In one embodiment, the terms "shape" and "form", as used herein in the context of a cylindrical form of the device, are used interchangeably.

The term "hollow cylindrical form", as used herein, preferably relates to the form of a cylinder that is empty in the inside, particularly that has a void in the inside, and has an internal radius and an external radius. The thickness of a device having a substantially hollow cylindrical form relates to the difference between the external radius and the internal radius of the substantially hollow cylinder. The shape formed at the ends of a hollow cylinder are typically rings, particularly annular rings. In one embodiment, the device is in the form of a hollow cylinder, and the channel of the device is the void of the hollow cylinder. The term "open cylindrical form", as used herein, preferably relates to the form of a cylinder open at both ends. In one embodiment, the term "open cylinder", "hollow cylinder", and "tube" are used interchangeably. In one embodiment, said cylindrical form is a hollow cylindrical form and/or an open cylindrical form. In one embodiment, the device is in the form of a tube. In one embodiment, the device, e.g. in the form of a tube, has an opening such as a cutting line along the longitudinal axis of said device.

The term "channel", as used herein in the context of the device, preferably relates to a void of the device of the invention which allows blood flow through said device. In one embodiment, the channel is a straight channel from an open end of the device to another open of the device. In one embodiment, e.g. if the device comprises a porous matrix, the channel comprises two or more interconnected channels. The term "internal channel", as used herein, preferably relates to a channel which extends along the longitudinal axis of the device. In one embodiment, the channel is a lumen. In one embodiment, the channel, preferably internal channel, comprises or consists of a lumen. Preferably, a lumen is the cavity, particularly channel, within a tube or tubular structure, such as within a hollow cylinder. In one embodiment, the terms "channel", "internal channel", and "lumen" are used interchangeably. In one embodiment, the device comprises a lumen; wherein, preferably, said lumen is configured to allow blood flow from upstream of said device through said lumen of said device to downstream of said device. In one embodiment, the terms "upstream" and "downstream" relate to "upstream" and "downstream", respectively, of said device with respect to the blood flow through said blood vessel; e.g. "upstream" relates to the blood flow of said blood vessel upstream of said implanted device and "downstream" relates to the blood flow of said blood vessel downstream of said implanted device.

In one embodiment, the polymeric matrix does not comprise pores having a diameter ≥ 20 µm, preferably ≥ 15 µm. In one embodiment, the polymeric matrix does not comprise pores. By providing the device without pores or with pores < 20 µm, a substantially physiological blood flow can be achieved, particularly bleeding flow through the polymeric matrix (instead of through the channel) is prevented. In one embodiment, the device is configured such that blood flows through the channel of the device, and that blood does not flow through the polymeric matrix.

In one embodiment, the channel has a radius in a range of from about 1 µm to about 5.9 mm, preferably in a range of from about 0.4 mm to about 3.9 mm, more preferably in a range of from about 0.9 mm to about 3.4 mm, even more preferably in a range of from about 1.3 mm to about 3 mm and/or a length in a range of from about 0.25 mm to about 80 mm, preferably in a range of from about 0.5 mm to about 50 mm, more preferably in a range of from about 1 mm to about 25 mm, even more preferably in a range of from about 3 mm to about 20 mm.

In one embodiment, said device is configured to release said active agent into said channel, if said device is contacted with a fluid, particularly blood. Advantageously, when the device is implanted in a blood vessel and is contacted with the blood flowing through the channel of the device, the active agent is released into the target tissue, particularly target tissue downstream of said device. For example, the active agent is released into the blood flowing through the channel and then reaches the target tissue directly downstream of the device.

The term "to allow blood flow through said device", as used herein, preferably relates to enabling a blood flow through said device in a manner that corresponds to a substantially physiological blood flow; particularly to a blood flow, such as a laminar and/or turbulent blood flow, substantially corresponding to the physiological blood flow through the respective blood vessel in which the device is positioned. In one embodiment, a channel configured to allow blood flow through said device has a suitable diameter and optionally suitable surface properties to enable a substantially physiological blood flow through the device, particularly through the channel of the device.

The term "elastically deformable", as used herein, preferably relates to the ability of the device of the invention to return to its original shape and size when the influence(s) or force(s) causing the deformation, such as a pulse of the blood system or a balloon used to position the device of the invention, are removed. For example, elastically deformable relates to a temporary change in length, volume, or shape of said device by a stress such as an inflated balloon or a pulse, particularly by a stress that is less than an elastic limit of the device. In one embodiment, each of the layers of the device is elastically deformable. In one embodiment, the device of the invention has an elastic modulus in a range of from about 1 kPa to about 100 MPa. In one embodiment, the device is elastically deformable in a direction substantially perpendicular to a longitudinal axis of said device. In one embodiment, the device is elastically deformable in a radial direction of said device, and optionally in a longitudinal direction of said device. For example, the elastic deformability of the device may be due to the material properties of the device, e.g. due to the polymeric matrix being elastically deformable, and/or may be due to the geometric properties of the device, e.g. due to the shape of the device such as a clasp shape, spiral shape, or reticulated shape. In one embodiment, the device has a clasp shape, spiral shape, or reticulated shape. In one embodiment, at least a part of said device is elastically deformable. In one embodiment, at least a part along a circumferential direction of said device is elastically deformable. For example, at least a half of the device along a circumferential direction of the device, e.g. in the form of a tube, is elastically deformable.

In one embodiment, the device has a longitudinal axis and a transverse axis; wherein, preferably, said channel is oriented along said longitudinal axis. In one embodiment, the device, particularly the cylindrical form, has an inner radius and an outer radius; wherein, preferably, a thickness of said device is the difference between the outer radius and the inner radius. In one embodiment, a length of said device is a length along said longitudinal axis. In one embodiment, the terms "aligned along" and "oriented substantially parallel to", particularly in the context of an alignment of fibers, are used interchangeably.

The term "biocompatible polymeric matrix", as used herein, relates to a polymeric matrix which is not harmful or toxic to animals, particularly vertebrates, such as humans. In one embodiment, the biocompatible polymeric matrix comprises or consists of biocompatible polymer(s); and optionally any of fibers, nanoparticles, a hydrogel, and combinations thereof.

The term "polymeric matrix", as used herein, preferably relates to a structure, particularly a solid such as a three-dimensional solid, comprising or consisting of one or more polymers. In one embodiment, the polymeric matrix is a layer of the device comprising one or more polymers and optionally comprising an active agent. In one embodiment, the polymeric matrix comprises one or more polymers in the form of fibers and/or hydrogel(s). Preferably, the term "fibers", as used herein, relates to nanofibers and/or microfibers. For example, the polymeric matrix may comprise fibers in an amount in a range of from 30% to 90% by volume. Advantageously, fibers reinforce the polymeric matrix and thus enhance the stability of the device. In one embodiment, the terms "polymeric matrix" and "polymeric scaffold" are used interchangeably. In one embodiment, the device and/or the polymeric matrix do/does not comprise any pores having a diameter of more than 1 µm, preferably of more than 100 nm, even more preferably of more than 50 nm. In one embodiment, the polymeric matrix does not comprise any interconnected pores. In one embodiment, the device comprises more than one layer; wherein, optionally, each layer of said more than one layer comprises a polymeric matrix. In one embodiment, the device has at least two layers, wherein the first layer has a first polymeric matrix and the second layer has a second polymeric matrix, wherein the first polymeric matrix and the second polymeric matrix are the same or different. For example, the first polymeric matrix and the second polymeric matrix may be different in a polymer composition and/or a density, such as a polymeric density. For example, an inner layer of the device may have a first polymeric matrix, e.g. a poly(lactic-co-glycolic acid) matrix or polycaprolactone matrix, which has a density that is lower than a density of a second polymeric matrix, e.g. a poly(lactic-co-glycolic acid) matrix or polycaprolactone matrix, forming an intermediate layer or an outer layer of the device. In one embodiment, the polymeric matrix and/or the device comprises a mineral and/or an organic substance. In one embodiment, the polymeric matrix and/or the device comprises said biocompatible polymeric matrix and a mineral.

The term "biocompatible polymer", as used herein, relates to a polymer which is not harmful or toxic to animals, particularly vertebrates, such as humans. Particularly, biocompatible polymers are not harmful or toxic to living tissue, organs, bodies, and organisms such as animals. In one embodiment, the biocompatible polymer is selected from poly(lactic-co-glycolic acid), poly(lactic acid), polycaprolactone, poly(glycolic acid), poly(glycerol sebacate), polycaprolactam, elastin, fibrillin, fibrullin, poly(1-acrylonitrile), poly(vinyl alcohol), polygluconate, polyglycolide, dextran, type I collagen, type II collagen, type IV collagen, elastin, silk fibroin, polymandelide, poly(trimethylene carbonate), polydioxanone, poly(4-hydroxybutyrate), poly(butylene succinate), polyphosphazenes, polyanhydrides, polyphosphoesters, polyoxalate, silica gel, alginate, polyethylene glycol, poly(2-oxazoline), gelatin, polyglycidol, polyurethane, and combinations thereof; wherein, preferably, said biocompatible polymer is selected from poly(lactic-co-glycolic acid), poly(lactic acid), polycaprolactone, poly(glycolic acid), poly(glycerol sebacate), and combinations thereof; wherein, more preferably, said biocompatible polymer is poly(lactic-co-glycolic acid), polycaprolactone, or a combination thereof. When referring to "combinations thereof' in the context of polymers, such a "combination" may relate to a copolymer thereof. In one embodiment, the expression "combinations thereof, as used herein in the context of polymers, is meant to be understood to include copolymers thereof. In one embodiment, the expressions "combinations thereof' and "combinations and copolymers thereof' are herein used interchangeably in the context of polymers. In one embodiment, said first biocompatible polymer, said second biocompatible polymer, and, if present, said third biocompatible polymer are the same biocompatible polymer or different biocompatible polymers. In one embodiment, the term "poly(lactic acid)", as used herein, relates to poly(L-lactide) (PLLA) and/or poly(DL-lactide). The device and/or the polymeric matrix may comprise random and/or block copolymers of the above polymers, such as poly(L-lactide-co-caprolactone). The biocompatible polymer is preferably biodegradable, more preferably bioresorbable.

The term "biodegradable", as used herein, preferably relates to the breakdown of the device when implanted in the body of a patient. The breakdown may comprise the breakdown of materials, such as the polymeric matrix, of the device, which may then be excreted and/or absorbed. For example, the polymeric matrix may degrade and the respective polymers may be excreted, and/or the active agent may be released and absorbed by the body. The term "bioresorbable", as used herein, preferably relates to the breakdown of the device and absorption of the components of the device when the device is implanted in the body of a patient. Advantageously, a biodegradable, particularly bioresorbable device does not need to be surgically removed. Accordingly, biodegradable, particularly bioresorbable devices enhance the comfort for the patient and prevent the risk associated with additional surgeries. In one embodiment, said device is biodegradable, preferably bioresorbable. In one embodiment, said device is biodegradable, preferably bioresorbable, at a temperature in a range of from about 4°C to about 43°C, preferably at a temperature in a range of from about 34°C to about 43°C. In one embodiment, said device is biodegradable, preferably bioresorbable, at body temperature. In one embodiment, said device is biodegraded, preferably bioresorbed, within a duration in a range of from about 10 min to about 600 days, preferably within a duration in a range of from about 30 min to about 300 days, more preferably in a range of from about 45 min to about 90 days, even more preferably in a range of from about 60 min to about 10 days. For example, a second or third layer comprising bortezomib may biodegrade, preferably bioresorb, within a duration in a range of from about 30 min to about 4 hours, and a first or second layer comprising dexamethasone may biodegrade, preferably bioresorb, within a duration in a range of from about 10 min to about 10 hours. Preferably, the third layer degrades prior to the second and first layer. Preferably, the second layer degrades prior to the first layer. Preferably, the device degrades from inside to outside, e.g. from an inner layer to an outer layer. In one embodiment, at least 95% of the device degrades within 90 days, preferably 60 days, after implantation.

The term "an active agent", as used herein, relates to any ingredient that provides an effect, such as a biologically active or other direct effect, in the diagnosis, cure, mitigation, treatment, or prevention of a disease, and/or affects the structure or any function of the body of humans or animals, particularly relates to an active pharmaceutical ingredient, preferably relates to a drug. The term "active agent", as used herein, preferably relates to an active agent selected from chemotherapeutic agents such as proteasome inhibitors, e.g. bortezomib, or anthracyclines, e.g. doxorubicin; immunosuppressants such as glucocorticoids, e.g. dexamethasone; calcium channel blockers such as nimodipine; antithrombotic agents such as tissue plasminogen activator, acetylsalicylic acid, phenprocoumon, dabigatran, apixaban, edoxaban, or heparin; antibodies such as anti-ICAM-1 antibodies, anti-MCH2 antibodies, or anti-VEGF antibodies; antihypertensive agents such as angiotensin-converting-enzyme inhibitors or beta blockers; matrix metalloproteinase inhibitors; cytokines such as interleukins; chemokines; Parkinson's disease drugs such as levodopa, dopamine agonists, or monoamine oxidase-B inhibitors; Huntington's disease drugs such as tetrabenazine, deutetrabenazine, haloperidol, fluphenazine, amantadine, levetiracetam, or clonazepam; and combinations thereof. In a preferred embodiment, the active agent is selected from chemotherapeutic agents such as proteasome inhibitors, e.g. bortezomib; immunosuppressants such as glucocorticoids, e.g. dexamethasone; calcium channel blockers such as nimodipine; antibodies such as anti-ICAM-1 antibodies, anti-MCH2 antibodies, or anti-VEGF antibodies; and combinations thereof; wherein, optionally, said active agent is selected from bortezomib, dexamethasone, nimodipine, an anti-ICAM-1 antibody, and combinations thereof. In a preferred embodiment, the active agent is selected from chemotherapeutic agents, preferably proteasome inhibitors, more preferably bortezomib, alone or in combination with a further active agent, preferably an immunosuppressant, more preferably a glucocorticoid, even more preferably dexamethasone. In one embodiment, the device comprises more than one layer, wherein at least one layer comprises an active agent. In one embodiment, the device comprises at least two layers, wherein at least two of said at least two layers comprise an active agent, wherein the active agent present in said layers are the same or different. For example, the device may comprise at least two layers, wherein one layer comprises a first active agent, for example a chemotherapeutic agent such as a proteasome inhibitor, and one layer comprises a second active agent, for example an immunosuppressant such as a glucocorticoid. In one embodiment, a chemotherapeutic agent is selected from proteasome inhibitors, preferably is bortezomib. In one embodiment, an immunosuppressant is selected from glucocorticoids, preferably is dexamethasone. In one embodiment, the terms "active agent", "active ingredient", and "drug" are used interchangeably. In one embodiment, the active agent is independently, particularly independently of its occurrence in a first, second, and/or third layer, selected from chemotherapeutic agents such as proteasome inhibitors, e.g. bortezomib; immunosuppressants such as glucocorticoids, e.g. dexamethasone; calcium channel blockers such as nimodipine; antithrombotic agents such as tissue plasminogen activator, acetylsalicylic acid, phenprocoumon, dabigatran, apixaban, edoxaban, or heparin; antibodies such as anti-ICAM-1 antibodies, anti-MCH2 antibodies, or anti-VEGF antibodies; antihypertensive agents such as angiotensin-converting-enzyme inhibitors or beta blockers; matrix metalloproteinase inhibitors; cytokines such as interleukins; chemokines; Parkinson's disease drugs such as levodopa, dopamine agonists, or monoamine oxidase-B inhibitors; Huntington's disease drugs such as tetrabenazine, deutetrabenazine, haloperidol, fluphenazine, amantadine, levetiracetam, or clonazepam; and combinations thereof. In one embodiment, the term "antithrombotic agent" relates to a thrombolytic agent, an antiplatelet agent, and/or an anticoagulant agent. An angiotensin-converting-enzyme inhibitor may be selected from benazepril, zofenopril, perindopril, trandolapril, captopril, enalapril, lisinopril, and ramipril. In one embodiment, the active agent is not an antibiotic such as vancomycin, is not aspirin, is not vascular endothelial growth factor (VEGF), is not fibroblast growth factor beta (b-FGF), is not stromal derived factor-1 alpha, and/or is not heparin. In one embodiment, the active agent is not vancomycin and/or aspirin. In one embodiment, the first layer comprises an antithrombotic agent such as tissue plasminogen activator, acetylsalicylic acid, phenprocoumon, dabigatran, apixaban, edoxaban, or heparin. In one embodiment, the outer layer comprises an active agent selected from antithrombotic agents such as tissue plasminogen activator, acetylsalicylic acid, phenprocoumon, dabigatran, apixaban, edoxaban, or heparin; wherein, preferably, the inner layer comprises an active agent other than said active agent of said outer layer, preferably an active agent other than an antithrombotic agent.

For example, highly advantageous release properties can be achieved when the active agent is provided as follows: In one embodiment, the active agent is embedded in said polymeric matrix, optionally is embedded in a hydrogel present in said polymeric matrix and/or is encapsulated in nanoparticles present in said polymeric matrix; said active agent is bound to said device, preferably to an inner surface of said device, preferably via a linker; and/or said active agent is coated onto an inner surface of said device, preferably onto a surface of said channel. Advantageously, by embedding the active agent in the polymeric matrix, the release of the active agent can be fine-tuned. The active agent may be directly embedded in the polymeric matrix, e.g. by mixing the active agent with a polymer solution for the polymeric matrix during the preparation of the device. Alternatively or additionally, the active agent may be embedded in a hydrogel which is part of the polymeric matrix and/or interspersed in the polymeric matrix. Advantageously, hydrogels enhance the mechanical properties of the device. Alternatively or additionally, the active agent may be encapsulated in nanoparticles, for example nanoparticles present in said polymeric matrix, e.g. mesoporous silica nanoparticles, poly(lactic-co-glycolic acid) nanoparticles, and/or inulin nanoparticles. Advantageously, nanoparticles allow for a controlled and fine-tuned release of said active agent. Alternatively or additionally, the active agent may be bound to said device, e.g., by covalent binding, preferably to an inner surface of said device, particularly to a surface of said channel. Advantageously, by binding the active agent to said device, even large active agents such as antibodies may be administered using the device of the invention. Alternatively or additionally, the active agent may be coated onto an inner surface of said device, preferably onto a surface of said channel, for example in the form of coating layer such as a third layer. Advantageously, a coating of said inner surface with said active agent allows for a facilitated release of said active agent into the bloodstream.

The term "configured to release said active agent into said channel", as used herein, preferably relates to properties that allow a release of the active agent into said channel, particularly that allow a release of the active agent into blood flowing through the channel when the device is positioned in a blood vessel of the patient. For example, the properties may include any of a suitable composition of the polymeric matrix, a suitable density of the polymeric matrix, and a suitable layering of the device. For example, the density of an inner layer may be lower than a density of an intermediate or outer layer, so that a release of the active agent from the inner layer into said channel, particularly into blood flowing through said channel, is facilitated.

In one embodiment, said device comprises nanofibers, preferably polymeric nanofibers. The term "polymeric nanofibers", as used herein, relates to nanofibers comprising or consisting of one or more polymers. In one embodiment, said nanofibers comprise silicon dioxide and/or titanium oxide. For example, said nanofibers may be polymeric nanofibers comprising silicon dioxide and/or titanium oxide. In one embodiment, said nanofibers comprise or consist of one or more biocompatible polymers, as defined herein. In one embodiment, said nanofibers comprise or consist of poly(lactic-co-glycolic acid), poly(lactic acid), polycaprolactone, poly(glycolic acid), poly(glycerol sebacate), polyurethane, silk fibroin, or a combination thereof, preferably comprise or consist of poly(lactic-co-glycolic acid), polycaprolactone, polyurethane, silk fibroin, or a combination thereof, more preferably comprise or consist of poly(lactic-co-glycolic acid), polycaprolactone, or a combination thereof. In one embodiment, said fibers such as microfibers comprise or consist of one or more biocompatible polymers, as defined herein. In one embodiment, said fibers such as microfibers comprise or consist of poly(lactic-co-glycolic acid), poly(lactic acid), polycaprolactone, poly(glycolic acid), poly(glycerol sebacate), polyurethane, silk fibroin, or a combination thereof, preferably comprise or consist of poly(lactic-co-glycolic acid), polycaprolactone, polyurethane, silk fibroin, or a combination thereof, more preferably comprise or consist of poly(lactic-co-glycolic acid), polycaprolactone, or a combination thereof. In one embodiment, said nanofibers are selected from poly(lactic-co-glycolic acid) nanofibers, polycaprolactone nanofibers, poly(lactic acid) nanofibers, poly(lactic acid) nanofibers comprising titanium oxide, silica gel nanofibers, and combinations thereof. In one embodiment, said biocompatible polymeric matrix comprises or consists of nanofibers and/or microfibers, preferably nanofibers. Advantageously, fibers have a reinforcing character and enhance the mechanical performance of the device. Nanofibers may be fibers with diameters in the nanometer range; typically with diameters between 1 nm and 1 µm. In one embodiment, the nanofibers have a diameter < 1000 nm. The term "fibers", as used herein, preferably relates to nanofibers and/or microfibers, preferably nanofibers.

In one embodiment, said fibers are aligned along a longitudinal axis of said device; said fibers are aligned at an angle in a range of from >0° to <180° to the longitudinal axis of said device; said fibers are aligned along a circumferential direction of said device; said fibers are aligned along a radial direction of said device; said fibers are oriented as a grid; and/or said fibers are randomly oriented. Advantageously, the alignment of the fibers can be adjusted to provide the desired properties, particular mechanical properties, in accordance with the respective blood vessel in which the device is to be positioned. If the device has more than one layer, the layers may have the same or a different nanofiber alignment. For example, fibers in a first layer may be aligned along a longitudinal axis of said device, and fibers in a second layer may be randomly oriented. For example, fibers in a first layer may be aligned along a longitudinal axis of said device, and fibers in a second layer may be aligned along a circumferential direction of said device. The grid may have any pattern. In one embodiment, the grid has a substantially hexagonal pattern, a substantially triangular pattern, or a substantially rectangular pattern. For example, the fibers may be prepared using electrospinning and/or melt electrowriting. The advantage of electrospinning and melt electrowriting is that the material, particularly the polymers, can be precisely shaped into fibers such as nanofibers and/or microfibers. An advantage of spinning is that even heat-labile active agents, such as antibodies, can be processed using spinning such as electrospinning. An advantage of melt electrowriting is that fibers such as microfibers can be precisely deposited into pre-defined shapes and it is a solvent-free process. For example, antibodies may be directly associated with fibers when preparing the fibers with spinning such as electrospinning.

In one embodiment, said device comprises nanoparticles, preferably mesoporous silica nanoparticles, poly(lactic-co-glycolic acid) nanoparticles, and/or inulin nanoparticles. In a preferred embodiment, said nanoparticles comprise said active agent. Advantageously, by incorporating, particularly encapsulating, the active agent in nanoparticles, the active agent is stabilized and protected. For example, the active agent may be stabilized and protected by said nanoparticles until the nanoparticle is released into said channel. Advantageously, when the active agent is incorporated in nanoparticles, the active agent release properties can be efficiently controlled and fine-tuned. In one embodiment, the device comprises nanoparticles comprising bortezomib and/or comprises nanoparticles comprising dexamethasone. For example, the device may comprise mesoporous silica nanoparticles comprising bortezomib. In one embodiment, mesoporous silica nanoparticles comprise poly(ethylene glycol)-block-poly(d,l-lactide). In a preferred embodiment, said nanoparticles comprise said active agent.

In one embodiment, said device comprises a hydrogel. Advantageously, a hydrogel enhances the mechanical properties of the device. Advantageously, the hydrogel enhances the active agent release properties of the device. In one embodiment, said hydrogel is configured to release said active agent, preferably to release said active agent into said channel, if said hydrogel is contacted with a fluid, particularly blood. In one embodiment, said hydrogel is interspersed between fibers of the polymeric matrix. For example, the polymeric matrix may comprise or consist of fibers, preferably nanofibers and/or microfibers, and hydrogel. For example, the fibers may be aligned in the form of a grid with the hydrogel being interspersed in the grid. In one embodiment, the hydrogel fills any pores present in the polymeric matrix, preferably such that the device does not comprise any pores.

In one embodiment, said hydrogel comprises poly(ethylene glycol), gelatin, poly(2-oxazoline), polyglycidol, silk fibroin, polyurethane, alginate, gelatin methacrylate, collagen, chitosan, hyaluronic acid, heparin, chondroitin sulfate, styrenated gelatin, synthetic extracellular matrix analogs, polyfumarate, phosphoester, PEGylated fibrinogen, poly(vinyl alcohol), poly(propylene fumarate), polypeptides, polyphosphazenes, poly(trimethylene carbonate), or any combination thereof. In a preferred embodiment, said hydrogel comprises poly(ethylene glycol), gelatin, alginate, gelatin methacrylate, collagen, or any combination thereof. In one embodiment, the hydrogel is a polyethylene glycol) hydrogel, a poly(2-oxazoline) hydrogel, a gelatin hydrogel, a polyglycidol hydrogel, a silk fibroin hydrogel, and/or a polyurethane hydrogel. In one embodiment, the hydrogel comprises or consists of a swellable material, such as poly(2-ethyl-2-oxazine). In one embodiment, the hydrogel is a swellable hydrogel.

The device of the invention may comprise more than one layer. For example, the device of the invention may comprise more than one layer of a polymeric matrix. In one embodiment, each layer comprises a polymeric matrix. In one embodiment, the device of the invention has two or more layers, such as three layers. Advantageously, the active agent can be released in a controlled and sustained manner with a device of the invention, particularly when the device comprises more than one layer.

In one embodiment, said device comprises a first layer and a second layer; optionally comprises a third layer; wherein, preferably, at least one of said first layer, said second layer, and, if present, said third layer, comprises said active agent. In one embodiment, said device comprises a first layer, a second layer, and a third layer.

In one embodiment, said first layer comprises a first biocompatible polymer, preferably a shape memory polymer and/or a swellable material; wherein, optionally, said first layer comprises fibers, nanoparticles, a hydrogel, an active agent, and/or a cell adhesion molecule. In one embodiment, said first layer is an outer layer of said device. In one embodiment, said first layer forms an outer surface of said device. In a preferred embodiment, the first layer comprises fibers, preferably nanofibers. Cell adhesion molecules are typically selected from cell surface proteins that are involved in the binding of cells with other cells or with the extracellular matrix. A cell adhesion molecule may be selected from integrine, claudine, desmosome, catechols, fibrin, thrombin, tannic acid, and a combination thereof. In a preferred embodiment, the first layer comprises poly(lactic-co-glycolic acid) and/or polycaprolactone, preferably polycaprolactone. In one embodiment, the first layer comprises a polymeric matrix comprising or consisting of poly(lactic-co-glycolic acid) and/or polycaprolactone, preferably polycaprolactone. In one embodiment, the terms "first layer" and "outer layer" are used interchangeably. In one embodiment, the first layer comprises fibers, particularly nanofibers, preferably fibers comprising polycaprolactone.

Advantageously, shape memory polymers and swellable materials enhance the form stability of the device. A shape memory polymer may comprise or consist of polycaprolactone, oligo(e-caprolactone), methyl methacrylate, polyethylene glycol) dimethacrylate, polyurethane, poly (D, L-lactide), poly(vinyl alcohol), ethylene vinyl acetate copolymer, and combinations thereof. In one embodiment, the first layer comprises said shape memory polymer and a further biocompatible polymer. In one embodiment, the first layer comprises a swellable material and optionally a biocompatible polymer. In one embodiment, the first layer comprises said shape memory polymer, preferably comprises polycaprolactone, and a swellable material, preferably poly(2-ethyl-2-oxazine). In one embodiment, the device comprises a swellable material, preferably poly(2-ethyl-2-oxazine). In one embodiment, a first layer, a second layer, and/or, if present, a third layer comprise(s) a swellable material, preferably poly(2-ethyl-2-oxazine). In one embodiment, the first layer comprises a swellable material, preferably poly(2-ethyl-2-oxazine). In one embodiment, a swellable material is poly(2-ethyl-2-oxazine). In one embodiment, the first layer comprises said polycaprolactone and poly(2-ethyl-2-oxazine). A first layer comprising polycaprolactone and optionally poly(2-ethyl-2-oxazine) exhibits advantageous properties both in terms of an efficient production of the device and in terms of in vivo use. For example, the first layer may be prepared using melt electrowriting. Advantageously, melt electrowriting allows for a precise formation of the 3D structure of the layer. Advantageously, a layer comprising poly(2-ethyl-2-oxazine) may swell when implanted in the blood vessel, thereby anchoring the device in the blood vessel.

In one embodiment, said second layer comprises a second biocompatible polymer, preferably poly(lactic-co-glycolic acid), polycaprolactone, poly(glycolic acid), or a combination thereof; wherein, optionally, said second layer comprises fibers, nanoparticles, a hydrogel, and/or an active agent. The first biocompatible polymer, the second biocompatible polymer, and, if present, the third biocompatible polymer are biocompatible polymers as defined herein. In one embodiment, said second layer comprises nanoparticles comprising said active agent and/or comprises fibers such as nanofibers. In one embodiment, said second layer comprises a second biocompatible polymer, preferably poly(lactic-co-glycolic acid), polycaprolactone, poly(glycolic acid), or a combination thereof; and an active agent such as an immunosuppressant; wherein, optionally, said second layer comprises fibers such as nanofibers, nanoparticles, and/or a hydrogel; wherein, preferably, said second layer comprises nanoparticles comprising said active agent. In one embodiment, the second layer is an inner layer of the device, if the third layer is absent, or is an intermediate layer of the device, if the third layer is present. In a preferred embodiment, the second layer comprises poly(lactic-co-glycolic acid), poly(glycolic acid), or a combination thereof; preferably comprises poly(glycolic acid). In one embodiment, the second layer comprises a polymeric matrix comprising or consisting of poly(lactic-co-glycolic acid), poly(glycolic acid), or a combination thereof; preferably poly(glycolic acid). In one embodiment, the device has two layers, wherein, preferably, each layer comprises a biocompatible polymeric matrix. In one embodiment, the device comprises a first layer, a second layer, and a third layer, wherein, after implantation, the second layer degrades when the third layer is partially or completely, preferably completely, degraded. In one embodiment, the device comprises a first layer, a second layer, and a third layer, wherein said second layer comprises a first active agent, and said third layer comprises a second active agent; wherein, preferably, said second active agent is released into said blood vessel prior to a release of said first active agent into said blood vessel. For example, said third layer may be an inner layer of said device comprising an active agent which shall be released first, and the second layer is an intermediate layer of said device comprising an active agent which shall be released second. Advantageously, thereby consecutive treatments may be provided with a single device.

In one embodiment, the inner layer, particularly the third layer or, if the third layer is absent, the second layer, comprises a biocompatible polymer, preferably a biocompatible polymer selected from poly(lactic-co-glycolic acid), poly(glycolic acid), poly(vinyl alcohol), polyoxalate, polyethylene glycol, poly(2-oxazoline), gelatin, polyglycidol, polyurethane, silk fibroin, silica gel, alginate, and combinations thereof. Advantageously, an inner layer comprising said biocompatible polymer(s) has highly advantageous active agent release properties. Preferably, the inner layer comprises an active agent, e.g. an active agent that shall be released prior to another active agent such as an active agent present in an intermediate layer or outer layer. In one embodiment, said third layer, if present, comprises said active agent, optionally comprises a third biocompatible polymer, fibers such as nanofibers, nanoparticles, and/or a hydrogel. In one embodiment, said third layer comprises nanoparticles comprising said active agent. In one embodiment, said third layer comprises a third biocompatible polymer, preferably poly(lactic-co-glycolic acid), polycaprolactone, poly(glycolic acid), or a combination thereof; and an active agent such as a chemotherapeutic agent; wherein, optionally, said third layer comprises fibers such as nanofibers, nanoparticles, and/or a hydrogel; wherein, preferably, said third layer comprises nanoparticles comprising said active agent. In one embodiment, the third layer comprises or consists of a coating comprising said active agent. In one embodiment, the third layer is a coating on the second layer, wherein the coating comprises or consists of the active agent. In one embodiment, when the device comprises a first layer, a second layer, and a third layer, the terms "third layer" and "inner layer" are used interchangeably. In one embodiment, when the device comprises a first layer and a second layer, but not a third layer, the terms "second layer" and "inner layer" are used interchangeably. In one embodiment, when the device comprises a first layer, a second layer, and a third layer, the terms "second layer" and "intermediate layer" are used interchangeably. In a preferred embodiment, the third layer comprises poly(lactic-co-glycolic acid), poly(glycolic acid), or a combination thereof; preferably comprises poly(glycolic acid). In one embodiment, the third layer comprises a polymeric matrix comprising or consisting of poly(lactic-co-glycolic acid), poly(glycolic acid), or a combination thereof; preferably poly(glycolic acid). In one embodiment, the device has three layers, wherein, preferably, each layer comprises a biocompatible polymeric matrix. Advantageously, a device comprising three layers is highly stable and allows to release one or more active agents in a controlled and sustained manner.

In a preferred embodiment, the first layer comprises polycaprolactone; the second layer comprises poly(lactic-co-glycolic acid), poly(glycolic acid), or a combination thereof, preferably poly(glycolic acid); and the third layer comprises poly(lactic-co-glycolic acid), poly(glycolic acid), or a combination thereof, preferably poly(glycolic acid). In one embodiment, the first layer and the second layer comprise fibers such as nanofibers, and the third layer comprises nanoparticles; wherein, optionally, the first layer, the second layer, and/or the third layer comprises a hydrogel. In one embodiment, the first layer and the second layer comprise fibers such as nanofibers, and the second layer and third layer comprise nanoparticles; wherein, optionally, the first layer, the second layer, and/or the third layer comprises a hydrogel. In one embodiment, the first layer comprises fibers such as nanofibers, and the second layer comprises nanoparticles; wherein, optionally, the third layer comprises nanoparticles; wherein, optionally, the first layer, the second layer, and/or the third layer comprises a hydrogel. In a preferred embodiment, said nanoparticles comprise said active agent.

In one embodiment, the device comprises an outer layer comprising fibers such as nanofibers, and comprises an inner layer comprising nanoparticles comprising active agent. In one embodiment, the device comprises a first layer comprising fibers such as nanofibers, and a second layer comprising nanoparticles comprising active agent; wherein, optionally, said first layer and/or said second layer comprises a hydrogel. In one embodiment, the device comprises an outer layer comprising fibers such as nanofibers, an intermediate layer comprising fibers such as nanofibers and/or nanoparticles, and an inner layer comprising nanoparticles comprising active agent; wherein, optionally, said first layer, said second layer, and/or said third layer comprises a hydrogel. In one embodiment, the device comprises an outer layer comprising fibers such as nanofibers, an intermediate layer comprising fibers such as nanofibers, and an inner layer comprising active agent; wherein, optionally, said first layer, said second layer, and/or said third layer comprises a hydrogel.

In one embodiment, in a radial direction, said first layer is arranged on top of said second layer; wherein, preferably, said second layer is arranged on top of said third layer, if present. In one embodiment, the first layer faces the endothelium of the blood vessel. In one embodiment, the second layer and the third layer, or the first layer and the second layer, comprise poly(lactic-co-glycolic acid), polycaprolactone, poly(glycolic acid), or a combination thereof; wherein, optionally, the outer layer has a higher density than the inner layer.

Advantageously, using different layers, particularly different polymeric matrices, that sequentially release different active agents, drugs can be administered in a controlled sequential manner even hours/days after removal of a stent retriever-wire-tube system or a balloon catheter system with which the device may be applied. Advantageously, two or more drugs may be sequentially released into the damaged tissue such as brain tissue.

In one embodiment, a density of said third layer is lower than a density of said second layer. In one embodiment, a density of said third layer is lower than a density of said second layer and said first layer. The term "density", as used herein, preferably relates to a substance's mass, particularly to a mass of a layer and/or a mass of a polymeric matrix, per unit of volume. Density may also refer to the packing density of the polymer(s) in a polymeric matrix, for example, the polymeric matrix of the layer. Without wishing to be bound by any theory, the inventors believe that the time period in which the layers dissolve depends on the density of the base substance, particularly of the polymeric matrix, in the respective layer. The device preferably dissolves from the inside, for example from the second or third layer, to the outside, for example to the first layer. Preferably, the density of the polymeric matrix is lower on the inside of the device than on the outside. In one embodiment, the molar mass of said third layer is lower than the molar mass of said second layer. In one embodiment, the molar mass of an inner layer is lower than the molar mass of an outer layer.

In one embodiment, said active agent of said second layer is different from said active agent of said third layer. In one embodiment, said active agent of said second layer is different from said active agent of said first layer. In one embodiment, said active agent of said third layer is different from said active agent of said first layer. In one embodiment, said active agent of said first layer, said active agent of said second layer, and said active agent of said third layer are different active agent. For example, one of said layers may comprise a chemotherapeutic agent, preferably a proteasome inhibitor, more preferably bortezomib, and another one of said layers may comprise an immunosuppressant, preferably glucocorticoid, more preferably dexamethasone. When referring to "said layers", said first layer, said second layer, and/or said third layer is/are meant. The device may comprise two or more layers, for example two layers, three layers, four layers, or more layers. In one embodiment, the device comprises four layers. Preferably, each of said layers comprises a polymeric matrix. In one embodiment, said device comprises more than one active agent, for example a combination of a chemotherapeutic agent, preferably a proteasome inhibitor, more preferably bortezomib, and an immunosuppressant, preferably glucocorticoid, more preferably dexamethasone. In one embodiment, said active agent is a combination of bortezomib and dexamethasone. In one embodiment, said first layer comprises a first active agent and said second layer comprises a second active agent. In one embodiment, said first layer comprises a first active agent, said second layer comprises a second active agent, and said third layer comprises a third active agent. In one embodiment, said second layer comprises a first active agent, and said third layer comprises a second active agent. The first active agent, the second active agent, and, if present, the third active agent are independently selected from the active agents defined herein. In one embodiment, said first active agent, said second active agent, and, if present, said third active agent are the same active agent or different active agent. In one embodiment, the active agent is coated onto an inner surface of said device. In one embodiment, the active agent is onto a first layer or a second layer to form a second layer or third layer, respectively. In one embodiment, the device comprises a first layer and a second layer, wherein the first layer comprises a first active agent and the second layer comprises a second active agent, wherein the first active agent and the second active agent are different active agents. In one embodiment, the device is coated with cell adhesion molecules on an outer surface of said device, preferably with cell adhesion molecule selected from integrins, claudins, desmosomes, catechols, fibrin, thrombin, tannic acid, and any combination thereof. In one embodiment, the device comprises two or more layers, wherein one layer comprises bortezomib and PLGA and one layer comprises dexamethasone and PLGA.

In one embodiment, said device has a length in a range of from about 0.25 mm to about 80 mm, preferably in a range of from about 0.5 mm to about 50 mm, more preferably in a range of from about 1 mm to about 25 mm, even more preferably in a range of from about 3 mm to about 20 mm. The length of the device is preferably a length from one end, preferably open end, of the device to another end, preferably open end, of the device, particularly a length along a longitudinal axis of the device. The terms "length" and "longitudinal extension", as used herein, may be used interchangeably. In one embodiment, said device has an opening along its length, such as a cutting line.

In one embodiment, said device has an outer radius in a range of from about 2 µm to about 6 mm, preferably in a range of from about 0.5 mm to about 4 mm, more preferably in a range of from about 1 mm to about 3.5 mm, even more preferably in a range of from about 1.4 mm to about 3 mm. Preferably, the device is in the form of a hollow cylinder with an outer radius in a range of from about 2 µm to about 6 mm. The outer radius is preferably the distance from the axial center of the device, particularly the longitudinal axis of the device, to an outer surface area of the device. For example, an outer radius of a device configured for A. cerebri media, particularly a M1 segment of the A. cerebri media, may be in a range of from about 1.4 mm to about 3 mm. In one embodiment, the device is configured to be implanted in the A. cerebri media, particularly in the M1 segment of the A. cerebri media.

In one embodiment, said device has an inner radius in a range of from about 1 µm to about 5.9 mm, preferably in a range of from about 0.4 mm to about 3.9 mm, more preferably in a range of from about 0.9 mm to about 3.4 mm, even more preferably in a range of from about 1.3 mm to about 3 mm. Preferably, the device is in the form of a hollow cylinder with an inner radius in a range of from about 1 µm to about 5.9 mm. Typically, the inner radius of a ring, tube or other hollow object is the radius of its cavity. Preferably, the inner radius is the radius of the channel of the device. For example, the inner radius may be the distance from the axial center of the device, particularly the longitudinal axis of the device, to an inner surface area of the device.

In one embodiment, said device has a thickness in a range of from about 1 µm to about 1 mm, preferably in a range of from about 20 µm to about 500 µm, preferably in a range of from about 50 µm to about 250 µm, even more preferably in a range of from about 75 µm to about 150 µm, e.g. about 100 µm. The term "thickness", as used herein in the context of the device, preferably relates to the difference between the outer radius and the inner radius of the device. For example, the thickness may relate to the thickness of the polymeric matrix and/or the thickness of the layer(s) of the device. For example, the thickness may be the combined thickness of the first, the second, and, if present, the third layer of the device. With the above specified length, outer radius, inner radius, and/or thickness, devices can be provided which are advantageous in terms of mechanical properties, stability, and/or active agent release properties. Advantageously, with the above specified length, outer radius, inner radius, and/or thickness, devices can be provided which are suitable for various blood vessels.

In one embodiment, said device comprises a cutting line, optionally a cutting line along a longitudinal axis of said device. The cutting line may be a result of the preparation process, for example a cutting line for facilitating the removal of the device from a spinning mandrel. Advantageously, the cutting line may facilitate the application of the device in the body, for example when applying the device in a blood vessel using a balloon. A device comprising a cutting line may easily be wrapped around medical equipment used to apply the device, such as a balloon, a needle, and/or a wire. In one embodiment, the terms "cutting line" and "opening" are used interchangeably. Preferably, the cutting line is an opening along the length of said device. In one embodiment, the cutting line is an opening along the longitudinal axis of said device, wherein, preferably, said opening has the length of the device.

In one embodiment, the device is provided with a protective cover, preferably a protective sheathing; wherein, optionally, said protective cover comprises an active agent. In one embodiment, the device is provided in the form of a kit comprising said device and a protective cover, preferably a protective sheathing; wherein, optionally, said protective cover comprises an active agent. The protective cover preferably covers a surface, particularly outer surface, of said device. The protective sheathing preferably sheaths the device, particularly sheaths a longitudinal extension of said device. In one embodiment, the protective cover, particularly protective sheathing, comprises an active agent. For example, the protective cover, particularly protective sheathing, may be configured to directly administer an active agent when the device is implanted in a blood vessel. The device covered by the protective cover may be administered using a stent retriever system and/or a balloon catheter system; optionally, the protective cover may be removed with the stent retriever system and/or the balloon catheter system directly after implantation of the device.

Preferably, the device is for use in a method of preventing or treating a neurological disease, an oncological disease, a cardiological disease, a vascular disease, an immunological disease, a carcinoid, an infectious disease, and/or an edema. In one embodiment, said neurological disease is a brain disease and/or neurodegenerative disease. In one embodiment, said brain disease is selected from stroke, particularly ischemic stroke, subarachnoid hemorrhage, and basal ganglia diseases. In one embodiment, said neurodegenerative disease is Parkinson's disease. In one embodiment, said oncological disease is a cancer, preferably a solid cancer, more preferably a solid cancer selected from liver cancer, kidney cancer, colorectal cancer, osteosarcoma, pancreas cancer, brain cancer, and melanoma. The oncological disease may also be a carcinoid. In one embodiment, said cardiological disease is a heart disease, preferably myocardial infarction. In one embodiment, said vascular disease is an ischaemic disease, preferably peripheral vascular disease. In one embodiment, said carcinoid is a neuroendocrine tumor, such as a slow-growing type of neuroendocrine tumor originating in the cells of the neuroendocrine system. In one embodiment, said immunological disease is graft-versus-host disease. In one embodiment, said edema is a brain edema, preferably an edema associated with an ischemic stroke. The infectious disease may be any infectious disease. For example, the device may be applied to a blood vessel using a stent retriever system and/or a balloon catheter system. In one embodiment, the device is for use in a method of preventing or treating ischemic stroke and/or an edema, particularly an edema associated with an ischemic stroke; e.g. a device comprising bortezomib and/or an immunosuppressant such as dexamethasone, for example a device comprising a layer comprising bortezomib and a layer comprising dexamethasone. In one embodiment, the device is for use in a method of preventing or treating a subarachnoid hemorrhage; e.g. a device comprising a calcium channel blocker, preferably nimodipine.

The device may be applied to enhance the efficiency of a CAR-T cell therapy, particularly to enhance a cancer therapy. For example, the efficiency of the CAR-T cell therapy can be enhanced by influencing the microenvironment and activating CAR-T cells in the area of a tumor; e.g., by increasing the release of proinflammatory cytokines in blood vessels supplying the tumor. In one embodiment, the term "patient", as used herein, relates to a human or an animal, preferably a human.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.

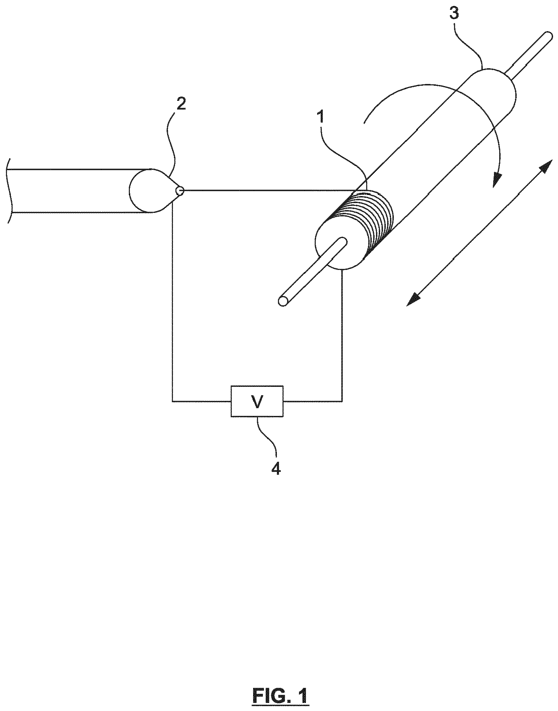

**Figure 1** shows an exemplary set-up for preparing a device of the invention, particularly an electrospinning device which prepares fibers (1) for the polymeric matrix of the device. The active agent may be incorporated in the polymer composition used to prepare the fibers or the active agent may be incorporated in the device after preparing the fibers for the polymeric matrix. The needle (2) releases the polymer composition which is deposited on the rotating mandrel (3) to form a nanofiber. The rotating mandrel rotates so that the fibers are aligned to form an substantially cylindrical device, e.g. having a circumferential alignment of fibers. A voltage (4) is applied. For example, the diameter of the rotating mandrel may be 3.5 mm to obtain a device with an advantageous radius.

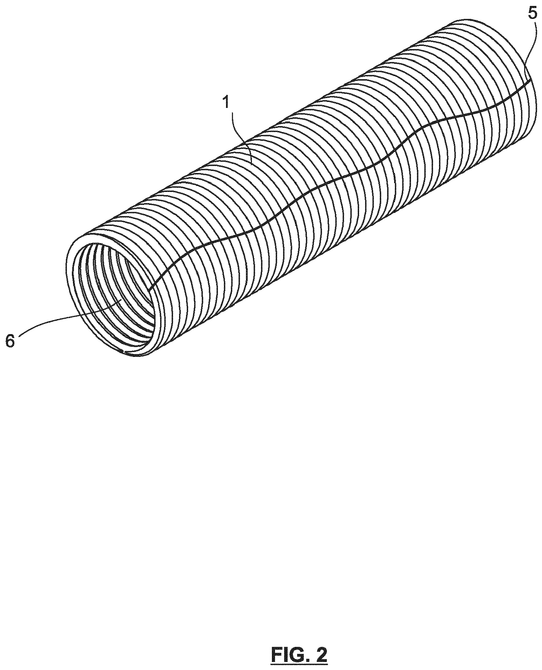

**Figure 2** shows an exemplary device of the invention comprising circumferentially aligned fibers (1) and a cutting line (5) along a longitudinal axis of the device. For example, as depicted, the polymeric matrix of the device may comprise or consist of fibers (1) such as fibers aligned along a circumferential direction of said device. The device may comprise a cutting line (5), for example if expedient in terms of manufacturing of the device and/or in terms of an application of the device in the blood vessel such as by using a balloon. The device comprises a channel (6), particularly internal channel, allowing for blood flow through the device.

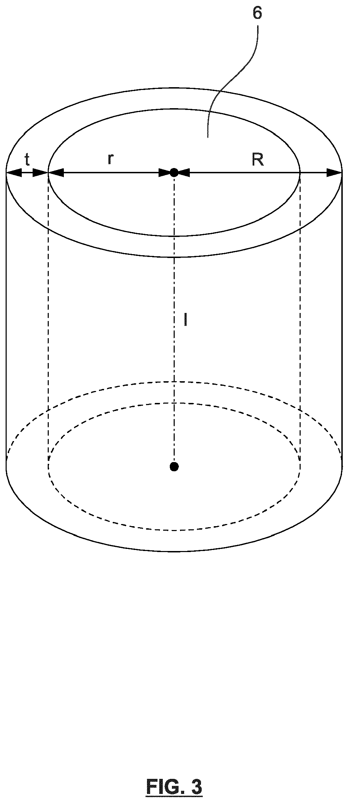

**Figure 3** shows an exemplary device of the invention having a substantially cylindrical form and a channel (6). The device has a thickness (t), an inner radius (r), and outer radius (R), and a length (l).

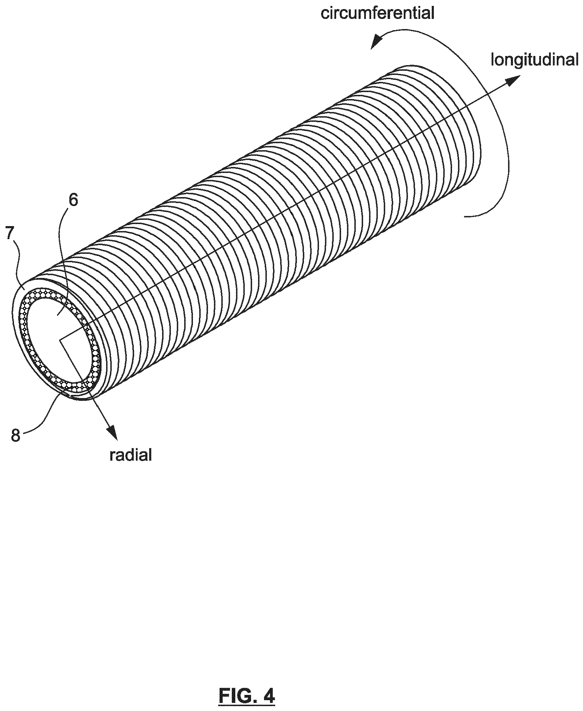

**Figure 4** shows an exemplary device of the invention comprising a first layer (7) comprising circumferentially aligned fibers (i.e. aligned along a circumferential direction of said device) and a second layer (8) comprising nanoparticles. The nanoparticles may incorporate the active agent. The first layer (7) and/or the second layer (8) may further comprise a hydrogel. The device of the invention has a circumferential direction, a longitudinal direction, and a radial direction.

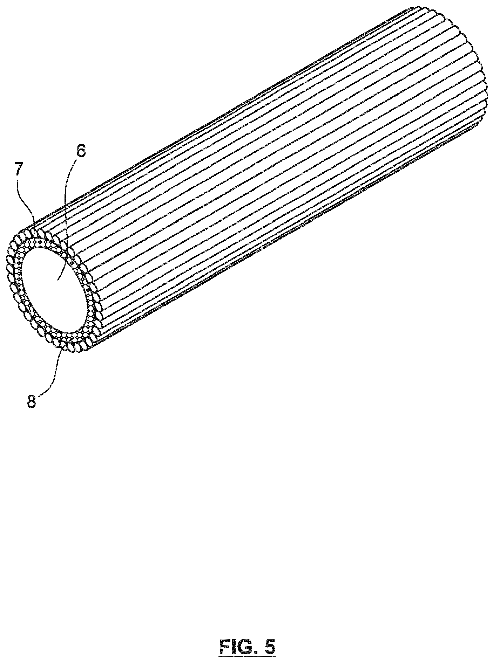

**Figure 5** shows an exemplary device of the invention comprising a first layer (7) comprising longitudinally aligned fibers (i.e. aligned along a longitudinal axis of said device) and a second layer (8) comprising nanoparticles and/or longitudinally aligned fibers. The nanoparticles may incorporate the active agent. The first layer (7) and/or the second layer (8) may further comprise a hydrogel.

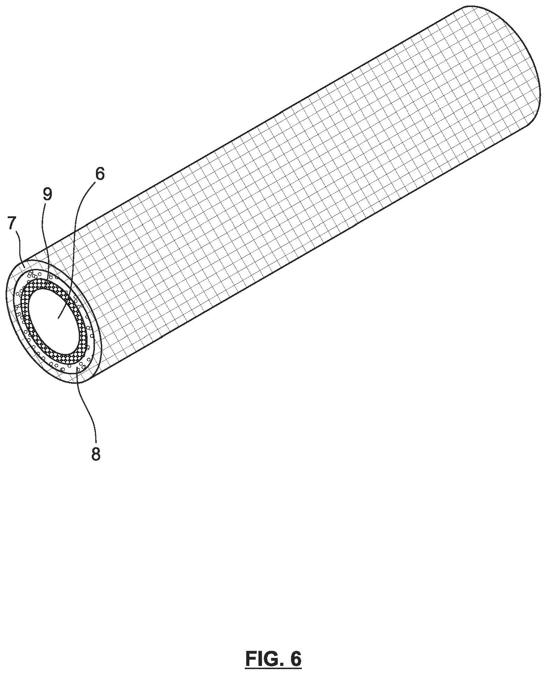

**Figure 6** shows an exemplary device of the invention comprising a first layer (7) comprising fibers which are oriented as a grid, a second layer (8) comprising nanoparticles, and a third layer (9) comprising nanoparticles and/or longitudinally aligned fibers. For example, the active agent may be encapsulated by the nanoparticles. The first layer (7), the second layer (8), and/or the third layer (9) may further comprise a hydrogel.

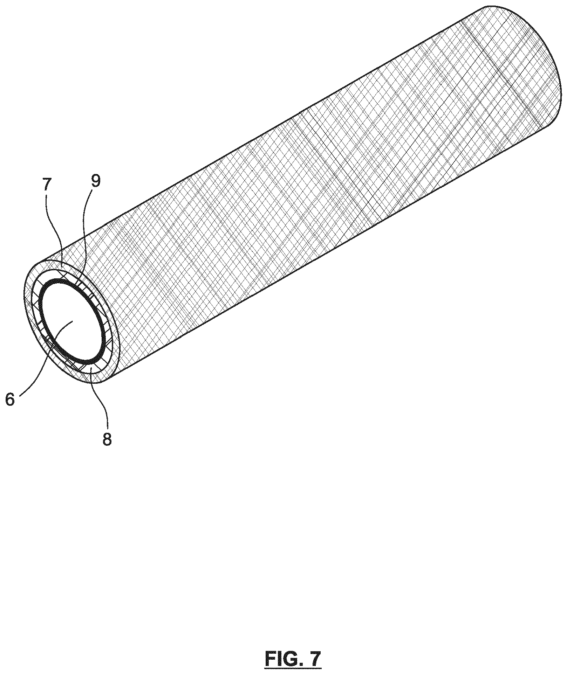

**Figure 7** shows an exemplary device of the invention comprising a first layer (7) comprising fibers which are randomly oriented, a second layer (8) comprising fibers which are randomly oriented, and a third layer (9) comprising or consisting of an active agent. For example, an active agent may be incorporated in the second layer (8) and the same or a different active agent may be comprised by the third layer (9). For example, the second layer (8) may have a lower density than the first layer (7).

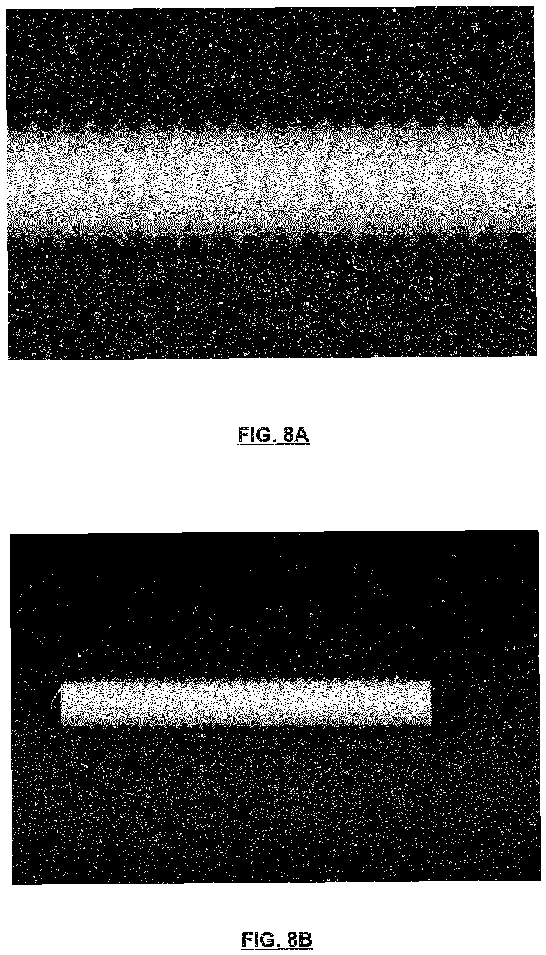

**Figure 8** shows photos of an exemplary device of the invention. The device shown in the photos (A, B) comprises electrospun randomly oriented fibers with diameters of around 2 micrometers and precisely aligned and stacked melt electrowritten fibers with a diameter of 15 micrometers. The inner diameter of the device is 3 mm. The length of the device is 20 mm.

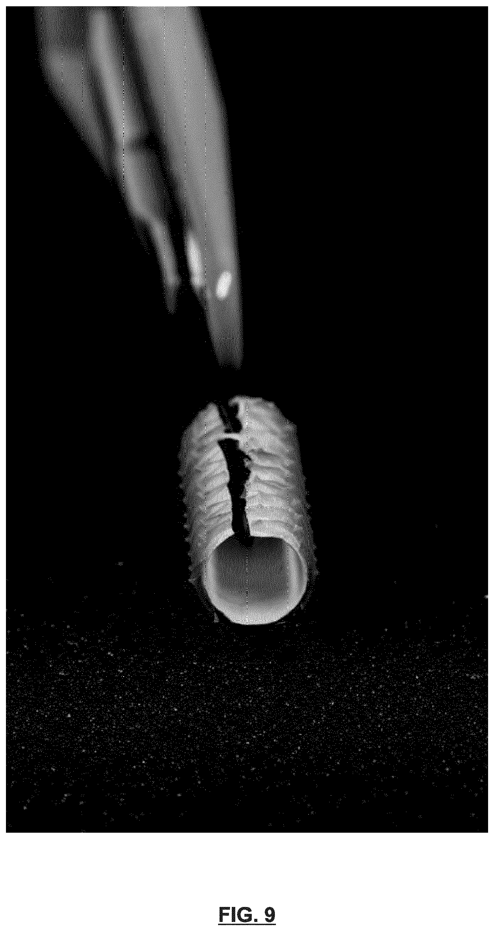

**Figure 9** shows a photo of an exemplary device of the invention with an opening along the longitudinal axis of the device.

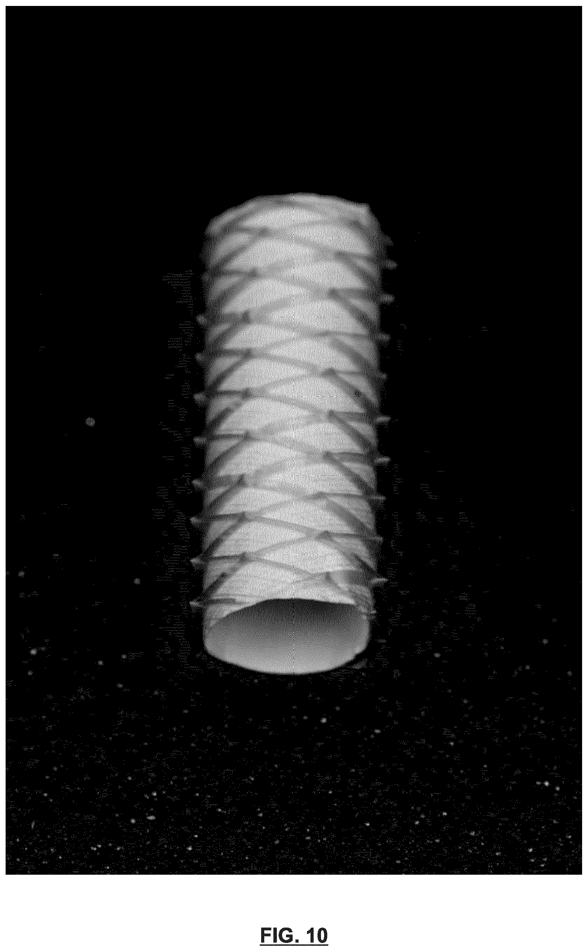

**Figure 10** shows a photo of an exemplary device of the invention with fibers which are oriented as a grid.

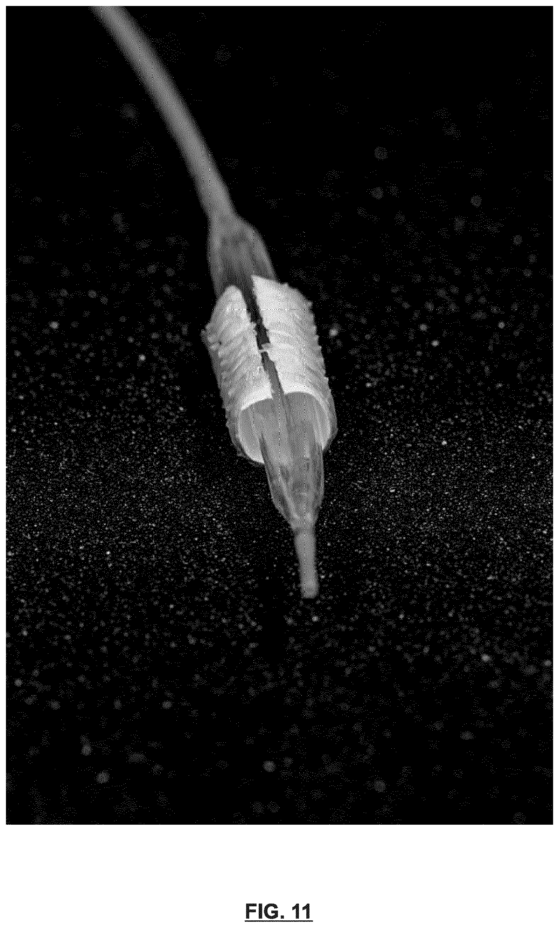

**Figure 11** shows a photo of an exemplary device of the invention with fibers which are oriented as a grid and with an opening along the longitudinal axis of the device.

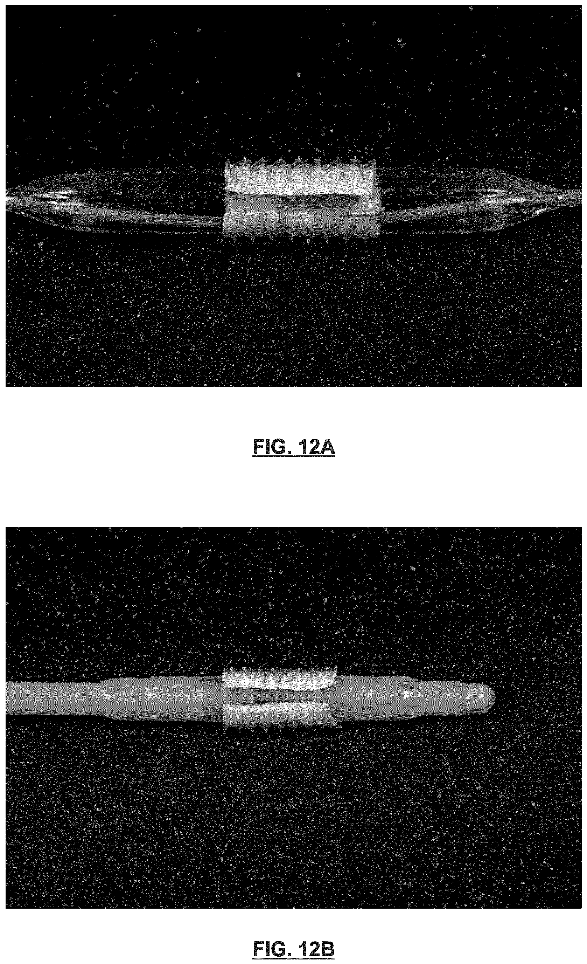

**Figure 12** shows photos (A, B) of an exemplary device of the invention.

In the following, reference is made to the examples, which are given to illustrate, not to limit the present invention.

### EXAMPLES

### Example 1: Preparation of device

A polymer, such as poly(lactic-co-glycolic acid) (PLGA) or poly(caprolactone) (PCL), and the active agent, particularly bortezomib, are dissolved in an organic solvent, such as 1,1,1,3,3,3-hexafluoro-2-propanol or chloroform, or an aqueous solvent. For example, a PLGA polymer having a lactide:glycolide ratio of 50:50 is used. The obtained solution of polymer and active agent is electrospun into nanotubes or printed into a hollow cylindrical form using a 3D printer.

### Example 2: Preparation of device

Step 1: A polymer, such as poly(lactic-co-glycolic acid) (PLGA), and the active agent, bortezomib, are dissolved in an organic solvent, such as 1,1,1,3,3,3-hexafluoro-2-propanol or chloroform. Other organic solvents are also suitable. For example, a PLGA polymer having a lactide:glycolide ratio of 50:50 is used. Other ratios are also suitable. The obtained solution of polymer and active agent is electrospun into nanofibers, which gather around a rotating target until they form a thin, hollow tube.

Step 2: Following step 1, a polymer, such as PLGA, and the active agent, dexamethasone, are dissolved in an organic solvent, such as 1,1,1,3,3,3-hexafluoro-2-propanol or chloroform. As stated above, other organic solvents are also suitable for any of the polymers used. The obtained solution of polymer and active agent is electrospun into nanofibers, which gather around the thin hollow tube, which is spun around the rotating target in step 1. During Steps 1 and 2 the target keeps rotating. Following step 2, the electrospun nanotube comprises two layers.

Step 3: Around the two layers, which are spun around the rotating target, another layer can be printed e.g. by means of melt electrowriting. This outer layer provides further stability and elasticity. It comprises polycaprolacton (PCL) combined with hydrophilic poly(2-ethyl-2-oxazine) (PEtOzi). By using the technique of melt electrowriting we can precisely determine the shape of the outer layer.

Step 4: Following that, the exemplary three-layered device can be cut into a reticulated shape, e.g. by a laser or a microkeratome. Alternatively, the three-layered device can be cut into a spiral shape, e.g. by a laser or a microkeratome.

### Example 3: Preparation of device

Step 1: A thin polycaprolacton (PCL)-layer is 3D-printed upon a rotating target by means of melt electrowriting.

Step 2: A polymeric matrix, comprising 3D structures ("walls" e.g. 36 µm) comprising PCL, which are perpendicular to the longitudinal axis of the rotating target, are printed upon the first layer by means of melt electrowriting with empty spaces in between.

Step 3: Another thin PCL-layer is printed on top of the polymeric matrix, particularly on top of the 3D structures e.g. walls.

Step 4: A liquid comprising blood plasma, proinflammatory cytokines and CAR-T-cells is optionally injected into the empty spaces.

### Example 4: Preparation of device

Step 1: A polymer, such as PLGA, and an active agent, a matrix metalloproteinase inhibitor, are dissolved in a solvent.

Step 2: Following that, the obtained solution of PLGA and MMP inhibitor is electrospun into nanofibers, which gather around a rotating target, until they form a thin, hollow tube.

Step 3: A thin polycaprolacton (PCL)-layer is 3D-printed upon the same rotating target by means of melt electrowriting.

Step 4: 3D structures ("walls") comprising PCL, which are perpendicular to the longitudinal axis of the rotating target, are printed upon the layer by means of melt electrowriting with empty spaces in between.

Step 5: Another thin PCL-layer is printed on top of the polymeric matrix, particularly on top of the 3D structures e.g. walls.

Step 6: A liquid comprising blood plasma, proinflammatory cytokines, nutrient solution and CAR-T-cells is optionally injected into the empty spaces.

### Example 5: Analysis of prepared devices

### Nanofiber analysis

The microstructure of the devices is examined by means of microscopy, such as scanning electronic microscopy, optical coherence tomography, or fluorescence microscopy. The average fiber diameter is determined from microscopy images, e.g. fluorescence images or SEM micrographs, by measuring about 20 to 60 randomly selected fibers.

### Mechanical characterization

The devices are analyzed with respect to their mechanical characteristics following the standard ISO/DIN 7198 "Cardiovascular implants-Tubular vascular prostheses". Advantageously, the device of the invention is elastically deformable in a radial direction so that the device may adapt to the blood flow and blood vessel.

### Example 6: Preparation of various exemplary devices

### Preparation of device (Ex. 1)

A polymer, such as poly(lactic-co-glycolic acid) PLGA or poly(caprolacotone) PCL, and the active agent, particularly bortezomib, are dissolved in an organic solvent, such as HFIP or chloroform. PLGA with a lactide:glycolide ratio of 50:50 can be used but different ratios can also be applied. The obtained solution of polymer and active agent is electrospun and collected onto a cylindrical collector that is rotating and can translate to prepare hollow cylindrical solution electrospun layers.

### Preparation of device (Ex. 2)

Step 1: A polymer, such as poly(caprolacotone) PCL, and the active agent, particularly bortezomib, are dissolved in an organic solvent, such as HFIP or chloroform. The obtained solution of polymer and active agent is electrospun and collected onto a cylindrical collector that is rotating to prepare hollow cylindrical solution electrospun layers.

Step 2: Around the solution electrospun layer, which is spun around the rotating target, another layer can be prepared from PCL using melt electrowriting. The deposition pattern of this layer can be used to influence the mechanical properties of the device. Size and thickness of this layer can be precisely adjusted during the printing process. This layer can be filled with a hydrogel containing active agents.

### Prototype based on Ex. 2:

Images of a prototype of Ex. 2 are shown in Figure 8 - Figure 12. This construct is composed of electrospun randomly oriented fibers with diameters of around 2 micrometers and precisely aligned and stacked melt electrowritten fibers with a diameter of 15 micrometers. The inner diameter of the tube is 3 mm. The length of the as produced tube is 20 mm. The size can be adjusted by cutting with a scalpel. Both layers are produced from PCL and the construct was cut open with a scalpel. For the generation of the inner layer, solution electrospinning was used. Medical grade PCL was dissolved in HFIP and transferred to a syringe with a blunt tip. A high voltage of 12 kV was applied to the tip. The distance from collector to spinneret was 12 cm. The material was extruded with 0.5 ml/h. A cylindrical collector with a diameter of 3 mm was used to collect the fibers. The collector was rotated at 120 rpm. After electrospinning, the mandrel was transferred to the melt electrowriting device. Deposition of the precisely oriented melt electrowritten fibers was achieved via a computer-controlled movement of the collector and by combining rotation and translation. Medical grade PCL was placed into a heating reservoir and heated to 89°C to melt the materials and process it. It was extruded from the reservoir with 0.7 bar and a high voltage of 4.5 kV was applied to the tip of the collector. After processing the tubular construct was manually removed from the collector and trimmed with a scalpel. The cut along the cutting line parallel to the main axis of the tube, was also made with a scalpel.

### Preparation of device (Ex. 3)

Step 1: A polymer, such as poly(lactic-co-glycolic acid) PLGA or poly(caprolacotone) PCL, and the active agent, particularly bortezomib, are dissolved in an organic solvent, such as HFIP or chloroform. PLGA with a lactide:glycolide ratio of 50:50 can be used, but different ratios can also be applied. The obtained solution of polymer and active agent is electrospun and collected onto a cylindrical collector that is rotating and can translate to prepare hollow cylindrical solution electrospun layers.

Step 2: A second layer of material is electrospun onto the layer prepared in step 1. This second layer can have a different or the same content of active agent and different PLGA with a changed lactide:glycolide ratio. In this step, co-spinning of PLGA and PCL can be used to generate a gradient of PLGA to PCL transition. PCL content can increase and/or decrease towards the outside of the layer generated in step 2.

Step 3: Around the two layers, which are spun around the rotating target, another layer can be prepared from PCL using melt electrowriting. The deposition pattern of this layer can be used to influence the mechanical properties of the device. Size and thickness of this layer can be precisely adjusted during the printing process. This layer can be filled with a hydrogel containing active agents.

Step 4: Another layer of electrospun fibers covers the previous layers.

Step 5: Following that, the exemplary four-layered device can be cut into a reticulated shape, e.g. by Laser or with a blade.

### Preparation of device (Ex. 4)

Step 1: A thin layer of polymer, such as poly(caprolacotone) PCL, is dissolved in an organic solvent, such as HFIP or chloroform. The obtained solution of polymer is electrospun and collected onto a cylindrical collector that is rotating and can translate to prepare hollow cylindrical solution electrospun layers. This layer allows diffusion of the active component but is impenetrable for silica particles.

Step 2: Another polymer layer can be prepared from PCL using melt electrowriting on top of the first one. The deposition pattern of this layer can be used to influence the mechanical properties of the device. Size and thickness of this layer can be precisely adjusted during the printing process. This layer is filled with a hydrogel containing active agents. Alternatively it can be filled with hydrogel and drug loaded and drug releasing silica particles.

Step 3: A thin layer of polymer, such as poly(caprolacotone) PCL, are dissolved in an organic solvent, such as HFIP or chloroform. The obtained solution of polymer is electrospun and collected onto a cylindrical collector that is rotating and can translate to prepare hollow cylindrical solution electrospun layers.

Step 4: Following that, the exemplary layered device can be cut into a reticulated shape, e.g. by Laser or with a blade.

### Preparation of device (Ex. 5)

Step 1: A thin layer of polymer, such as poly(caprolacotone) PCL, is dissolved in an organic solvent, such as HFIP or chloroform. The obtained solution of polymer is electrospun and collected onto a cylindrical collector that is rotating and can translate to prepare hollow cylindrical solution electrospun layers. This layer allows diffusion of the active component but is impenetrable for silica particles.

Step 2: Another polymer layer can be prepared from PCL using melt electrowriting on top of the first one. The deposition pattern of this layer can be used to influence the mechanical properties of the device. Size and thickness of this layer can be precisely adjusted during the printing process. This layer is filled with a hydrogel containing active agents. Alternatively it can be filled with hydrogel and drug loaded and drug releasing silica particles.

Step 3: A thin layer of polymer, such as poly(caprolacotone) PCL, are dissolved in an organic solvent, such as HFIP or chloroform. The obtained solution of polymer is electrospun and collected onto a cylindrical collector that is rotating and can translate to prepare hollow cylindrical solution electrospun layers.

Step 4: Following that, the exemplary layered device can be cut into a reticulated shape, e.g. by Laser or with a blade.

### Analysis of prepared devices (Ex. 6)

### Analysis of electrospun fibers:

The layers of the device are examined by means of microscopy such as scanning electron microscopy, optical coherence microscopy, or fluorescence and confocal microscopy. The thickness of the layer and the fiber diameter is analyzed based on images. 60 randomly chosen fibers are analyzed and the diameter is quantified by automated software analysis (e.g. imageJ scripts).

### Analysis of melt electrowritten fibers:

The microstructure of the layers of the device is examined by means of microscopy such as scanning electron microscopy, optical coherence microscopy, or fluorescence and confocal microscopy. The thickness of the layer and the fiber diameter is analyzed based on images. 20 randomly chosen fibers are analyzed and the diameter is quantified by automated software analysis (e.g. imageJ scripts). The quality of the deposited patterns is quantified based on images taken from the layers with regard to homogeneity of fiber deposition.

### Mechanical characterization:

The devices are analyzed with respect to their mechanical properties. Longitudinal and circumferential tests are performed in static and cyclic conditions. In addition, analysis following the standard ISO/DIN 7198 "Cardiovascular implants-Tubular vascular prostheses" can be performed.

### Biocompatibility tests:

Biocompatibility of the materials and the combined device is analyzed via direct and indirect methods. Standardized protocols and test methods are used.

### Analysis of drug release (Ex. 7)

Part 1: Analysis of release of test defined materials to determine diffusion of different layers The layers are tested regarding their permeability or diffusion for test substances with defined molecular weight. Commercially available Fluoresceinisothiocyanat-Dextran (FITC-Dextran) with different molecular weights can be used as fluorescent label to quantify permeability or diffusion.

### Part 2: Analysis of release of test active agents to determine diffusion of different layers

The layers are tested regarding their permeability or diffusion for active components. Permeability and release are analyzed with UV/Vis Spectroscopy, Mass spectroscopy of high-performance liquid chromatography.

### Part 3: Analysis of in vitro drug release

In vitro drug release is analyzed with in cell culture conditions using UV/Vis Spectroscopy, Mass spectroscopy of high-performance liquid chromatography. The influence of the released active agents is analyzed in an indirect cytocompatibility assay and with cell culture methods using marker and gene expression.

### Example 7: Analysis of in vitro drug release

In vitro release of the active agent from the device is characterized using a suitable method such as high-performance liquid chromatography or UV spectrophotometry.

### Example 8: Analysis of in vivo drug release

The in vivo drug release characteristics are examined by implanting the device in a blood vessel, such as an Arteria cerebri media or a blood vessel of corresponding size, of an animal, such as a mouse, rabbit, or rat. For example, the device is implanted in a blood vessel by means of a stent retriever system and/or a balloon catheter system.

The features of the present invention disclosed in the specification, the claims, and/or in the accompanying figures may, both separately and in any combination thereof, be material for realizing the invention in various forms thereof.

### REFERENCE SIGNS

| | |
|---|---|
| fibers | (1) |
| needle | (2) |
| rotating mandrel | (3) |
| voltage | (4) |
| cutting line | (5) |
| channel | (6) |
| first layer | (7) |
| second layer | (8) |
| third layer | (9) |

## Claims

1. A device for endovascular drug delivery, preferably an implant for endovascular drug delivery,
wherein said device has a substantially cylindrical form;
wherein said device comprises a channel, preferably an internal channel; wherein, preferably, said channel is configured to allow blood flow through said device;
wherein said device is elastically deformable, preferably elastically deformable in a direction substantially perpendicular to a longitudinal axis of said device;
wherein said device comprises a biocompatible polymeric matrix comprising a biocompatible polymer; and
wherein said device comprises an active agent;
wherein, preferably, said device is biodegradable;
wherein, preferably, said device is configured to release said active agent into said channel.

2. The device according to claim 1, wherein said biocompatible polymer is selected from poly(lactic-co-glycolic acid), poly(lactic acid), polycaprolactone, poly(glycolic acid), poly(glycerol sebacate), polycaprolactam, elastin, fibrillin, fibrullin, poly(1-acrylonitrile), poly(vinyl alcohol), polygluconate, polyglycolide, dextran, type I collagen, type II collagen, type IV collagen, elastin, silk fibroin, polymandelide, poly(trimethylene carbonate), polydioxanone, poly(4-hydroxybutyrate), poly(butylene succinate), polyphosphazenes, polyanhydrides, polyphosphoesters, polyoxalate, silica gel, alginate, polyethylene glycol, poly(2-oxazoline), gelatin, polyglycidol, polyurethane, and combinations thereof;
wherein, preferably, said biocompatible polymer is selected from poly(lactic-co-glycolic acid), poly(lactic acid), polycaprolactone, poly(glycolic acid), poly(glycerol sebacate), and combinations thereof;
wherein, more preferably, said biocompatible polymer is poly(lactic-co-glycolic acid), polycaprolactone, or a combination thereof.

3. The device according to claim 1 or 2, wherein said device comprises fibers, preferably nanofibers and/or microfibers, such as polymeric nanofibers; wherein, optionally, said fibers, preferably polymeric nanofibers, comprise silicon dioxide and/or titanium oxide;
wherein, preferably, said device comprises nanofibers, preferably selected from poly(lactic-co-glycolic acid) nanofibers, polycaprolactone nanofibers, poly(lactic acid) nanofibers, poly(lactic acid) nanofibers comprising titanium oxide, silica gel nanofibers, and combinations thereof.

4. The device according to claim 3, wherein said fibers are aligned along a longitudinal axis of said device; said fibers are aligned at an angle in a range of from >0° to <180° to the longitudinal axis of said device; said fibers are aligned along a circumferential direction of said device; said fibers are aligned along a radial direction of said device; said fibers are oriented as a grid; and/or said fibers are randomly oriented.

5. The device according to any one of the foregoing claims, wherein said device comprises nanoparticles, preferably mesoporous silica nanoparticles, poly(lactic-co-glycolic acid) nanoparticles, and/or inulin nanoparticles.

6. The device according to any one of the foregoing claims, wherein said device comprises a hydrogel;
wherein, optionally, said hydrogel is configured to release said active agent, preferably to release said active agent into said channel, if said hydrogel is contacted with a fluid, particularly blood.

7. The device according to any one of the foregoing claims, wherein said device comprises a first layer and a second layer; optionally comprises a third layer;
wherein said first layer comprises a first biocompatible polymer, preferably a shape memory polymer; wherein, optionally, said first layer comprises nanofibers, nanoparticles, a hydrogel, an active agent, and/or a cell adhesion molecule;
wherein said second layer comprises a second biocompatible polymer, preferably poly(lactic-co-glycolic acid) or polycaprolactone; wherein, optionally, said second layer comprises nanofibers, nanoparticles, a hydrogel, and/or an active agent;
wherein said third layer, if present, comprises an active agent, optionally comprises a third biocompatible polymer, nanofibers, nanoparticles, and/or a hydrogel;
wherein at least one of said first layer, said second layer, and, if present, said third layer, comprises said active agent.

8. The device according to any one of the foregoing claims, wherein said device comprises a first layer, a second layer, and a third layer;
wherein said first layer comprises a first biocompatible polymer, preferably a shape memory polymer;
wherein said second layer comprises a second biocompatible polymer, preferably poly(lactic-co-glycolic acid) or polycaprolactone, and an active agent such as an immunosuppressant; wherein, optionally, said second layer comprises nanofibers, nanoparticles, and/or a hydrogel; wherein, preferably, said second layer comprises nanoparticles comprising said active agent;
wherein said third layer comprises a third biocompatible polymer, preferably poly(lactic-co-glycolic acid) or polycaprolactone, and an active agent such as a chemotherapeutic agent; wherein, optionally, said third layer comprises nanofibers, nanoparticles, and/or a hydrogel; wherein, preferably, said third layer comprises nanoparticles comprising said active agent;
wherein, optionally, a density of said third layer is lower than a density of said second layer;
wherein, optionally, said active agent of said second layer is different from said active agent of said third layer.

9. The device according to any one of the foregoing claims, wherein said cylindrical form is a hollow cylindrical form and/or an open cylindrical form.

10. The device according to any one of the foregoing claims, wherein said device has a length in a range of from about 0.25 mm to about 80 mm, preferably in a range of from about 0.5 mm to about 50 mm, more preferably in a range of from about 1 mm to about 25 mm, even more preferably in a range of from about 3 mm to about 20 mm,
an outer radius in a range of from about 2 µm to about 6 mm, preferably in a range of from about 0.5 mm to about 4 mm, more preferably in a range of from about 1 mm to about 3.5 mm, even more preferably in a range of from about 1.4 mm to about 3 mm,
an inner radius in a range of from about 1 µm to about 5.9 mm, preferably in a range of from about 0.4 mm to about 3.9 mm, more preferably in a range of from about 0.9 mm to about 3.4 mm, even more preferably in a range of from about 1.3 mm to about 3 mm, and/or
a thickness in a range of from about 1 µm to about 1 mm, preferably in a range of from about 20 µm to about 500 µm, preferably in a range of from about 50 µm to about 250 µm, even more preferably in a range of from about 75 µm to about 150 µm, e.g. about 100 µm.

11. The device according to any one of the foregoing claims, wherein said device comprises a cutting line, optionally a cutting line along a longitudinal axis of said device.

12. The device according to any one of the foregoing claims, wherein said device is biodegradable, preferably bioresorbable,
wherein, optionally, said device is biodegradable, preferably bioresorbable, at a temperature in a range of from about 34°C to about 43°C.

13. The device according to any one of the foregoing claims, wherein said active agent is embedded in said polymeric matrix, optionally is embedded in a hydrogel present in said polymeric matrix and/or is encapsulated in nanoparticles present in said polymeric matrix; said active agent is bound to said device, preferably to an inner surface of said device, preferably via a linker; and/or said active agent is coated onto an inner surface of said device, preferably onto a surface of said channel.

14. The device according to any one of the foregoing claims, wherein said active agent is selected from chemotherapeutic agents such as proteasome inhibitors, e.g. bortezomib, or anthracyclines, e.g. doxorubicin; immunosuppressants such as glucocorticoids, e.g. dexamethasone; calcium channel blockers such as nimodipine; antithrombotic agents such as tissue plasminogen activator, acetylsalicylic acid, phenprocoumon, dabigatran, apixaban, edoxaban, or heparin; antibodies such as anti-ICAM-1 antibodies, anti-MCH2 antibodies, or anti-VEGF antibodies; antihypertensive agents such as angiotensin-converting-enzyme inhibitors or beta blockers; matrix metalloproteinase inhibitors; cytokines such as interleukins; chemokines; Parkinson's disease drugs such as levodopa, dopamine agonists, or monoamine oxidase-B inhibitors; Huntington's disease drugs such as tetrabenazine, deutetrabenazine, haloperidol, fluphenazine, amantadine, levetiracetam, or clonazepam; and combinations thereof;
wherein, preferably, said active agent is selected from chemotherapeutic agents such as proteasome inhibitors, e.g. bortezomib; immunosuppressants such as glucocorticoids, e.g. dexamethasone; calcium channel blockers such as nimodipine; antibodies such as anti-ICAM-1 antibodies, anti-MCH2 antibodies, or anti-VEGF antibodies; and combinations thereof; wherein, optionally, said active agent is selected from bortezomib, dexamethasone, nimodipine, an anti-ICAM-1 antibody, and combinations thereof;
wherein, more preferably, said active agent is selected from chemotherapeutic agents, preferably proteasome inhibitors, e.g. bortezomib.

15. A device according to any one of the foregoing claims for use in a method of preventing or treating a neurological disease, an oncological disease, a cardiological disease, a vascular disease, an immunological disease, a carcinoid, an infectious disease, and/or an edema;
wherein, preferably, said neurological disease is a brain disease and/or neurodegenerative disease, wherein, preferably, said brain disease is selected from stroke, particularly ischemic stroke, subarachnoid hemorrhage, and basal ganglia diseases, and wherein, preferably, said neurodegenerative disease is Parkinson's disease;
wherein, preferably, said oncological disease is a cancer, preferably a solid cancer, more preferably a solid cancer selected from liver cancer, kidney cancer, colorectal cancer, osteosarcoma, pancreas cancer, brain cancer, and melanoma;
wherein, preferably, said cardiological disease is a heart disease, preferably myocardial infarction;
wherein, preferably, said vascular disease is an ischaemic disease, preferably peripheral vascular disease;
wherein, preferably, said immunological disease is graft-versus-host disease;
wherein, preferably, said edema is a brain edema.
